# EUROPÄISCHE PATENTSCHRIFT

(11) **EP 0 673 913 B1**
(45) Veröffentlichungstag und Bekanntmachung des Hinweises auf die Patenterteilung: **10.02.1999**
(21) Anmeldenummer: 95104030.2
(22) Anmeldetag: 18.03.1995
(51) Int. Cl.: C07C 59/265, A61K 31/19

(54) **Calcium-Alkalicitratverbindungen und deren Verwendung als Arzneimittel**
Calcium-alkali-citrate compounds and their use as medicaments
Sels de citrates de calcium et d'alcalins ainsi que leur utilisation comme médicaments

(30) Priorität: 23.03.1994 DE 4409948; 23.03.1994 DE 4409949
(43) Veröffentlichungstag der Anmeldung: 27.09.1995
(73) Patentinhaber: MADAUS AG, D-51109 Köln (DE)
(72) Erfinder: Carcasona, Alfons, Dr. rer.nat, D-51065 Köln (DE); Reinhardt, Klaus, Prof. Dr. rer., D-48161 Münster (DE); Schwille, Paul-Otto, Prof. Dr. rer. nat. Dr. med, D-91080 Uttenreuth (Erlangen) (DE); Wächter, Wilfried, Dr. rer.nat., D-51469 Bergisch Gladbach (DE)

(56) Entgegenhaltungen:
- DE-A- 2 727 304
- MILCHWISSENSCHAFT, Bd. 39, Nr. 4, 1984 Seiten 195-197,
- CHEMICAL ABSTRACTS, vol. 102, no. 5, 1985 Columbus, Ohio, US; abstract no. 44346w, KARKLINS ET AL. 'production of citrates from n-alkanes by microbial synthesis.'
- ACTA RADIOLOGICA, Bd. 54, 1960 Seiten 305-315, B. CARLQVIST, A. NELSON 'Attempts to influence the elimination of radiostrontium.'

## Beschreibung

Die Erfindung betrifft die Herstellung und Verwendung von Calcium-Alkalimetallcitrat-Verbindungen als Arzneimittel.

Der Stand der Technik schlägt bei konsumierenden und degenerativen Krankheiten, bei denen ein stark kataboler Stoffwechsel auftritt, die Verabreichung von Hormonen wie Androgene vor. Der katabole Stoffwechsel äußert sich auch in einer negativen Stickstoffbilanz, wobei der Abbau von Proteinen, e.g. Muskelproteinen, durch Proteolyse erfolgt und die Protein-Biosyntheserate niedriger als die Eiweiß-Degradationsrate ist, sodaß das Verhältnis der Biosynthese- zur Abbaurate negativ ist. Das Auftreten von Tumoren, aber auch die Strahlen- und Zytostatikatherapiebehandlungen führen zu einem allgemeinen pathologischen Zustand der "Kachexie" ( = Auszehrung, Roche Lexikon Medizin Urban -Schwarzenberg, München-Wien-Baltimore, 2. Auflage) oder Atrophie des Organismus infolge tiefgreifender Stoffwechselstörungen von Organen und Geweben, was sich als starke Abmagerung und allgemeiner Kräfteverfall beispielsweise als Proteinschwund in der Muskulatur manifestiert.

Auch bei seniler oder Alterskachexie zeigt sich ein reduzierter Allgemeinzustand, der u. a. auch aufgrund kataboler Stoffwechsellage, wie stark erhöhter Proteinabbau, zu einem Eiweißmangelzustand führt.

Der Stand der Technik schlägt als palliative (krankheitsmildernde) Behandlung bei konsumierenden Erkrankungen, wie Tumorerkrankungen, bei Strahlen- und Zytostatikatherapien sowie in der Rekonvaleszenz die Verabreichung von Androgenen vor, um einen weiteren Kräfteverfall zu verhindern und den Aufbau lebenswichtiger Proteinreserven zu fördern, zumal z.B. die aromatischen Aminosäuren als Bausteine der Proteine nicht vom menschlichen Organismus selbst synthetisierbar sind. Die Applikation dieser Hormone führt zwar zu einer Positivierung der Stickstoffbilanz, wodurch die Proteinmasse, also auch die Muskelmasse, zunimmt und damit einhergehend das Körpergewicht im allgemeinen ansteigt. Jedoch sind die androgenen Nebenwirkungen sehr erheblich. Diese äußern sich durch Virilisierung und Menstruationsstörungen des weiblichen Organismus sowie Auftreten von Heiserkeit, rauchiger Stimme etc. bei männlichen Patienten. Gleichfalls sind bei Langzeittherapien mit Androgenen Veränderungen im Elektrolythaushalt zu beobachten, die schließlich zu Ödemen führen (Forth, Pharmakologie und Toxikologie, 2. Auflage, Wissenschaftsverlag Mannheim-Wien-Zürich, Seiten 325 -327).

So beschreiben Evans und Ivy (Journal of Applied Physiology, 1982, Band 52/6, Seiten 1643 - 1647), daß kastrierte Ratten eine Reduktion im Körpergewicht und eine Muskelatrophie erfahren, jedoch diese katabolische Wirkung der Kastration durch die Zugabe von Testosteronderivaten aufgrund ihrer antikatabolischen Effekte verhindert werden können. Alternative Mittel, welche keine androgenen Nebenwirkungen aufweisen, wurden nicht vorgeschlagen.

Darüberhinaus ist auch bei der verlängerten Kortikoidtherapie eine katabole Stoffwechsellage mit stark erhöhtem Proteinabbau zu diagnostizieren. Hierbei wird wie bei den oben beschriebenen Erkrankungen und Therapien versucht, durch Gabe von Androgenen die katabole Stoffwechsellage des Organismus einzuschränken und die antikatabolischen Wirkungen von Androgenen zur Verbesserung des Allgemeinzustandes des Organismus unter Inkaufnahme der oben beschriebenen Nebenwirkungen auszunutzen.

Auch sollte die durch katabolen Stoffwechsel bei Diabetes mellitus einhergehende Eiweißsynthesestörung mit der Einschmelzung von körpereigenem Protein (Muskelschwund) in Hinsicht auf den Eiweißverlust durch Verabreichung von Medikamenten, die eine antikatabolische Wirkung aufweisen, zumindest palliativ zusätzlich neben den herkömmlichen Therapien wie Insulingabe und diätetischer Behandlung verhindert werden (Heilmeyer, Innere Medizin, Springer-Verlag Berlin-Heidelberg-New York, 4. Auflage, Seiten 1069 und 1073).

Auch die zur allgemeinen Leistungssteigerung von Sportlern erfolgende Einnahme von männlichen Hormonen, wie Androgene, als Anabolika zum Aufbau von Muskelproteinen, welche zur Leistungssteigerung zwar beitragen, aber virilisierende Eigenschaften haben, ist abzulehnen und nicht empfehlenswert. Ein Mittel wäre erwünscht, welches im Gegensatz zu den Androgenen kein Hormon wäre, aber neben der zum Aufbau von Muskelproteinen, e.g. Muskelfasern, führenden antikatabolischen, anabolen Wirkungsweise auch gleichzeitig die Gewährleistung für die Abpufferung der bei hoher muskulärer Belastung, bei anaerober Glykolyse auftretenden Milchsäure bietet. Auch sollte das Mittel bei körperlicher Belastung der Sportler eine Normalisierung der bei Acidose auftretenden Kaliumungleichverteilung in intra- und extrazellulären Räumen ermöglichen und zu einer Reduzierung des bei Körperbelastung erhöhten respiratorischen Quotienten (Verhältnis von CO₂-Ausscheidung zu O₂-Aufnahme) führen, was zu einer ausgeglicheneren Verwendung von Sauerstoff bei hohen körperlichen Leistungsanforderungen führt.

Wie beschwerlich es ist, eine physiologische, muskuläre Leistungssteigerung zu erzielen, wird durch die Veröffentlichung von Powers et al. in European Journal of Applied Physiology 1990, Seiten 54 - 69 gezeigt. Sie berichten, daß keine Änderungen in der Herzfrequenz, im Plasmavolumen und Plasmakonzentration des Gesamtproteins durch Verabreichung von Getränken bei Belastung auftraten, welche Glukose und Elektrolyte (GP), oder nur Elektrolyte (EP) oder nur Wasser als Kontrolle (NEP) enthielten. Sie kommen zu dem Schluß, daß die durch körperliche Belastung hervorgerufenen Mißverhältnisse in den Blut-Elektrolyt- Konzentrationen durch die Zufuhr von Getränken somit nicht verringert werden können.

Weiterhin schlägt der Stand der Technik zur Therapie der Osteoporose die Verwendung einer Reihe von Medikamenten wie bestimmte Calciumverbindungen, Fluoride oder Östrogene vor, die jedoch aufgrund ihrer Nebenwirkungen auf den menschlichen Organismus, wie unten ausgeführt wird, nicht unbedenklich sind.

Calcium ist ein unentbehrlicher Bestandteil des Gewebes und der Organe, insbesondere des Skeletts, wobei 99,9% des Calciums sich im Skelett befindet, so daß bei Calciummangel oder aufgrund von altersabhängiger gesteigerter Empfindlichkeit gegen das endogen gebildete Parathormon, von Menopause oder von gestörter intestinaler Calciumresorption ein Knochenverlust auftritt. Die Verminderung, die eine knöcherne Brüchigkeit hervorruft, führt zu geringerer mechanischer Belastbarkeit und Neigung zu Frakturen und Spontanverformungen. Dieser als Osteoporose oder -porosis bekannte Verlust an Knochengewebe in knöchernen Organen wird beispielsweise therapiert durch die Verabreichung von bestimmten Calciumsalzen, wie Calciumglukonat oder früher durch Calciumchlorid, wobei jedoch, insbesondere bei der Applikation von Calciumchlorid, starke Nebenwirkungen auftreten.

Calciumglukonat wird herkömmlicherweise bei der Therapie von Hypocalcämie, bei Vitamin-D-Mangel und insbesondere bei der Immobilisations-(Alters-) Osteoporose als 10% Lösung injiziert oder als geringer konzentrierte Lösung mittels Dauerinfusion verabreicht. Dabei besteht jedoch die Gefahr der Hypercalcämie, wobei neben Arrhythmien ebenso aufgrund einer verminderten tubulären Resorption von NaCl und Flüssigkeit eine Polyurie resultieren kann (Forth et al., Pharmakologie und Toxikologie, 2. Auflage, Seiten 235 ff). Daher wird Calciumglukonat umständlicherweise auch bei oraler Einnahme möglichst unter Kontrolle verabreicht.

Das früher verabreichte CaCl₂ wird in der Regel nicht mehr appliziert, da irritierende Wirkungen auf Venen und den Gastrointestinaltrakt auftreten.

Darüber hinaus wird die Anwendung von Natriumfluorid vorgeschlagen, um aufgrund der Anregung der Knochenneubildung die verbliebenen Knochenstrukturelemente zu verstärken. Die Einnahme von Fluorid kann jedoch bei der Langzeiteinnahme zu Fluorose führen, so daß aufwendige und kostspielige jährliche Röntgenuntersuchungen erforderlich sind, um diese Nebenwirkungen zu vermeiden. Auch die vom Stand der Technik vorgeschlagene Kombination von Calcium und Vitamin D ist nicht frei von Nebenwirkungen, da plötzliche und starke gastrointestinale Beschwerden wie Magenschmerzen und Völlegefühl und damit einhergehend eine geringere Nahrungsaufnahme feststellbar sind (Bader et al., Lehrbuch der Pharmakologie und Toxikologie, 2. Auflage, Seiten 207 ff).

Außerdem muß bei der Einnahme von Fluoriden der Umstand berücksichtigt werden, daß häufig bereits dem Trinkwasser Fluoride zugesetzt werden, um möglichst Überdosierungen zu vermeiden. Auch die dem Stand der Technik entsprechende Kombination von Vitamin D und Natriumfluorid macht einen stationären Aufenthalt u.U. nötig, da Vitamin D-Intoxikation und Fluorose drohen können.

Weiterhin führen die früher empfohlenen Gaben von Östrogenen nur kurzfristig zu einer Besserung der Symptome der Osteoporose. Trotz der unzureichenden Therapiemöglichkeiten schlägt man bei älteren Patientinnen die Verabreichung von Östrogenen vor, obwohl das Risiko der Bildung von Korpuskarzinomen und Hyperplasien des Endometriums nicht zu vernachlässigen ist. Dabei kann außerdem der Mineralien- und Lipid-Spiegel im Serum ungünstig beeinflußt werden (laut MSD-Manual, 4. Auflage, Urban & Schwarzenberg).

Auch die Kombination von Natriumfluorid mit Calcium ist nicht unumstritten, zumal die Wirksamkeit dieser Behandlung nach Meinung der FDA noch nicht abschließend bewiesen ist (MSD-Manual, 4. Auflage, Urban & Schwarzenberg).

Da wegen der bisher unvollkommenen Therapiemöglichkeiten der Osteoporose, insbesondere der gezielten Therapie gegen den fortschreitenden Knochengewebeschwund und den Abbau der Strukturelemente keine erfolgversprechenden Medikamente zur Verfügung stehen, wird ein Therapieerfolg zur Zeit "nur durch Gaben von Natriumfluorid zu gewährleisten" sein (Heilmeyer,L., Innere Medizin, 4. Auflage, Springer-Verlag, Berlin), trotz der Nebenwirkungen, wie Hypotonie und Atemdepression, die vorwiegend aufgrund der Inhibierungseigenschaften von Fluoridderivaten auf viele Enzymsysteme hervorgerufen werden. In Ermangelung weiterer Heilmethoden verweist man auch auf die Anwendung konservativer Therapien wie Übungsbehandlung etc.

Eine Vielzahl von Calcium-organischen Verbindungen, welche in der pharmazeutischen Industrie als Calciumpräparate eine große Bedeutung haben, ist bekannt; insbesondere finden Calciumcitrat i.e. Ca₃(C₆H₅O₇)₂ als Tetrahydrat und Calciumcarbonat Anwendung. Das Calciumcitrat Ca₃(C₆H₅O₇)₂ ist in Wasser nur gering löslich und wird in Form von Injektionslösung, Kautablette, Dragee oder Lutschtablette bei Calciummangelerscheinungen, allergischen Reaktionen und Osteoporose appliziert. Calciumcarbonat wird als Antacidum häufig verabreicht, da seine Säurebindungskapazität im Vergleich zu Natriumhydrogencarbonat größer ist.

Weiterhin ist bekannt, daß ein saures Alkalicitrat der Formel K₆Na₆H₃(C₆H₅O₇)₅ als Hydrat zur Auflösung von Harnsäuresteinen und zur Verhinderung ihrer erneuten Bildung verabreicht wird. So beschreibt die DE PS 27 27 304 die Verwendung von diesem sauren Alkalicitrat zur Bekämpfung der Urolithiasis, wobei bei der Anwendung als Arzneimittel Citrat-, Natrium- und Kaliumionen in einem bestimmten äquivalenten Verhältnis freigesetzt werden und eine therapeutisch gewünschte pH-Wert-Erhöhung des Harns auf pH 6,2 bis 7,0 bewirkt wird. Bei Patienten mit Hyperurikusurie bewirkt saures Alkalicitrat, welches im Zustand eines definierten Kristallisats vorliegt, durch die gezielte pH-Anhebung im Harn eine Auflösung und zudem eine Verhinderung der Neubildung von Harnsäuresteinen.

Der Stand der Technik beschreibt zwar die Anwendung von Alkalicitraten und Citronensäure als Arzneimittel bei der Behandlung der oben genannten Krankheiten, dabei beschränken sich jedoch die bisher bekannten Therapien mit den Alkalicitraten ausschließlich auf Erkrankungen und o.g. Schädigungen der ableitenden Harnwege.

Aufgabe der Erfindung ist es, ein Mittel zur Verfügung zu stellen, das bei kataboler Stoffwechsellage zu applizieren ist, antikatabolisch die allgemeine Atrophie des Organismus hemmt und somit zu einem allgemeinen Protein- und Muskelproteinaufbau führt. Außerdem sollte es kein Hormon sein, welches die bei der Verabreichung von Androgenen zu beobachtende Virilisierung und die anderen Nebenwirkungen hervorruft. Das Mittel sollte somit zu einer Positivierung der Stickstoffbilanz führen und die bei konsumierenden und degenerativen Erkrankungen, bei Strahlen-, Zytostatikabehandlungen, seniler Kachexie sowie bei Niereninsuffizienz auftretende Proteinolyse unterdrücken und möglichst die Proteinbiosynthese fördern.

Zusätzlich ist es erforderlich, ein Mittel bereitzustellen, welches auch bei Langzeit-Kortikoidbehandlungen den Proteinabbau unterdrückt und die aufbauenden Stoffwechselvorgänge fördert und somit die Muskelproteinmasse erhöht.

Auch sollte das Mittel die Möglichkeit eröffnen, helfend in die Stoffwechsel lage des Organismus dermaßen einzugreifen, daß die Muskelmasse bei Leistungsportlern z.B. in Ruhezeiten zumindest gehalten oder sogar u.U. erhöht wird ohne die bei den Hormonen, wie Androgene, zu beobachtenden Nebenwirkungen.

Die Aufgabe der Erfindung besteht des weiteren darin, ein Mittel anzugeben, welches bei der Osteoporosebehandlung den fortschreitenden Knochengewebeschwund verhindert und möglichst die noch verbliebenen Strukturelemente im Knochen verstärkt, das sowohl für den menschlichen Organismus gut verträglich ist, i.e. eine komplikationslose Verstoffwechslung im menschlichen Organismus ermöglicht, als auch bei der Einnahme weiterer Medikamente keine Nebenwirkungen aufweist. Da man heutzutage aufgrund des wachsenden Kostendrucks in der Therapeutik weiterhin bestrebt ist, statt eine bereits manifeste Krankheit zu therapieren, einer zukünftigen Erkrankung vorzubeugen, sollte das Medikament nicht nur Knochensubstanz aufbauen helfen sondern auch auch bei möglichst rechtzeitiger Einnahme im Rahmen einer prophylaktischen Behandlung bereits den Abbau des Knochengewebes verhindern helfen, was gerade auch bei der Alters- oder bei der Steroidosteoporose erforderlich ist, da bekannterweise Cortikoide die Mobilisation von Calcium aus dem Skelett fördern.

Dazu ist ein Medikament nötig, welches keine Nebenwirkungen oder bei einer Langzeiteinnahme nur vernachlässigbare Nebenwirkungen aufweist und somit den Nierenstoffwechsel und den Mineralgehalt im Serum nicht beeinflußt. Da außerdem bei einer Langzeittherapie zur Behandlung altersbedingter Schwächezustände oder zur Prophylaxe von Osteoporose die Nierentätigkeit in erster Linie betroffen sein wird, ist gerade hierbei die Wirkung des zu verabreichenden Arzneimittels zu beachten.

Außerdem ist beachtenswert, daß das Medikament nicht nur die Darmausscheidung, die Sekretion sowie das Urinvolumen sondern auch die Nahrungsaufnahme nicht ungünstig beeinflussen darf, zumal gerade die Osteoporose als Involutionsosteoporose bei älteren Patientinnen/Patienten, die häufig unter großer Appetitlosigkeit leiden, als präsenile oder senile Form auftritt.

Ebenso ist es angebracht, die Wirksubstanz so zu wählen, daß sie einfach und damit preiswert herstellbar ist, um die Herstellungskosten des Medikaments zu senken und den Patienten ein preiswertes und wirksames Arzneimittel zur Verfügung zu stellen, was bei einer Langzeittherapie zur Prophylaxe von entscheidender Bedeutung ist.

Die Aufgabe wird gelöst durch die Verwendung von Calcium-Alkalimetallcitraten gemäß der Hauptansprüche und der Nebenansprüche. Die Unteransprüche betreffen bevorzugte Ausführungsformen dieses Erfindungsgegenstandes.

Gegenstand der Erfindung ist somit die Verwendung von mindestens einer Calcium-Alkalimetallcitrat-Verbindung der allgemeinen Formel CaXC₆H₅O₇, worin X ein Alkalimetall ist, oder eines Hydrates oder Hydratgemisches davon zum Aufbau von Muskelproteinen und zur Erhöhung der muskulären Stoffwechselleistung durch antikatabolische Stoffwechselwirkung.

Ein weiterer Gegenstand der Erfindung ist die Verwendung von mindestens einer Calcium-Alkalimetallcitrat-Verbindung der allgemeinen Formel CaXC₆H₅O₇, worin X ein Alkalimetall ist, oder eines Hydrates oder Hydratgemisches davon zur Rekonvaleszenz oder palliativen Behandlung bei kataboler Stoffwechsellage chronisch konsumierender oder degenerativer Krankheiten. Vorzugsweise kann man die Calcium-Alkalimetallcitrat-Verbindung der o.g. Formel bei Tumorkachexie, Niereninsuffizienz, seniler Kachexie, Alkoholabusus und Infekten verabreichen. Auch bei Strahlen- oder Zytostatikatherapie, verlängerter Kortikoidtherapie, postoperativen oder posttraumatischen Zuständen kann die o.g. Verbindung appliziert werden, wobei durch die antikatabolische Wirkung ein Proteinaufbau erfolgt und eine Reduzierung des Allgemeinzustandes des Organismus verhindert wird.

Ein weiterer Gegenstand der Erfindung ist die Verwendung von Calcium-Alkalimetallcitraten der Formel CaXC₆H₅O₇, worin X ein Alkalimetall ist oder eines Hydrats oder Hydratgemisches davon, zur Prophylaxe oder Behandlung des Knochengewebeschwundes und zur Verstärkung der Knochenstrukturelemente sowie zur Prophylaxe oder Behandlung der Osteoporose.

Besonders hat sich eine Ausführungsform als vorteilhaft erwiesen, welche dadurch gekennzeichnet ist, daß das Calcium-Alkalimetallcitrat in Form einer Mischung mit ein oder mehreren Zusatzstoffen verabreicht wird, wobei sich insbesondere als Zusatzstoff Citronensäure, gegebenenfalls im Überschuß, hervorragend eignet. Dabei zeigt sich, daß als Calcium-Alkalimetallcitrate CaNaC₆H₅O₇ und/oder CaKC₆H₅O₇ sich besonders gut eignen.

Ein weiterer Gegenstand der Erfindung betrifft ein Verfahren zur Herstellung der Calcium-Alkalimetallcitrate der Formel CaXC₆H₅O₇, worin X ein Alkalimetall ist, dadurch gekennzeichnet, daß man eine wäßrige Ca-Citrat-Suspension mit einem Alkalimetallcitrat und Citronensäure in konzentrierter wäßriger Lösung in einem Molverhältnis von Calciumcitrat, Alkalimetallcitrat und Citronensäure von 2:2:1 bis 2:2,5:1 bei einem pH-Bereich von 4,6 bis 4,8 bei einer Temperatur von 80-95°C unter Rückfluß während 1 bis 1,5 Stunden unter Rührung umsetzt und man entweder nach der Umsetzung die Lösung mit dem Niederschlag filtriert, den Rückstand mindestens einmal mit Wasser wäscht und trocknet oder man nach der Umsetzung die Lösung mit dem gebildeten Niederschlag sofort bis zur Trockne einengt, oder daß nach der Umsetzung die Lösung mit dem gebildeten Niederschlag als Suspension das Endprodukt ist.

In einer bevorzugten Ausführungsform vermischt man
a) eine wäßrige Suspension aus Calciumcitrat-Suspension mit 31,7 %(w/v) Calciumcitrat^{.}4H₂O mit einer wäßrigen Lösung, welche 0,56 m Alkalimetallcitrat und 0,28 m Citronensäure enthält,
b) rührt unter Wasserzugabe, vorzugsweise 1,5 Stunden lang, und arbeitet anschließend die Lösung bis zur Trocknung des Rückstandes wie oben angegeben auf.

In einer weiteren bevorzugten Ausführungsform verfährt man wie unter a) und b), verwendet aber eine wäßrige Lösung, welche 0,7 m Alkalimetallcitrat und 0,28 m Citronensäure enthält.

Weiterhin können Calcium-Alkalimetallcitrate der Formel CaXC₆H₅O₇, worin X ein Alkalimetall ist, dadurch hergestellt werden, indem man entweder Calciumcitrat mit einem Alkalimetallcarbonat, z.B. Kalium- oder Natriumcarbonat, und Citronensäure in konzentrierter wäßriger Lösung in einem Molverhältnis von Calciumcitrat, Alkalimetallcarbonat und Citronensäure von 2:3:3 bis 2:3,75:3 oder Calciumcitrat mit einem Alkalimetallhydrogencarbonat, z.B. Natrium- oder Kaliumhydrogencarbonat, oder Alkalimetallhydroxid, wie Natrium- oder Kaliumhydroxid, und Citronensäure in konzentrierter wäßriger Lösung in einem Molverhältnis von Calciumcitrat, Alkalimetallhydrogencarbonat bzw.-hydroxid und Citronensäure von 2:6:3 bis 2:7,5:3 bei einem pH-Bereich von 4,6 bis 4,8 bei einer Temperatur von 80-95°C unter Rückfluß während 1 bis 1,5 Stunden unter Rührung umsetzt und man entweder nach der Umsetzung die Lösung mit dem Niederschlag filtriert, den Rückstand mindestens einmal mit Wasser wäscht und trocknet oder man nach der Umsetzung die Lösung mit dem gebildeten Niederschlag sofort bis zur Trockne einengt, oder daß nach der Umsetzung die Lösung mit dem gebildeten Niederschlag als Suspension das Endprodukt ist.

Vorteilhaft ist es, daß man
a) eine wäßrige Suspension aus 31,7 % (w/v) Calciumcitrat^{.}4H₂O mit einer wäßrigen Lösung unter Rühren vermischt, welche 0,83 m Citronensäure enthält,
b) unter Wasserzugabe, vorzugsweise 1,5 Stunden lang, rührt,
c) Alkalimetallcarbonat bis zu einer Konzentration von 0,28 m zugibt,
d) die Lösung, vorzugsweise 0,5 Stunden lang, rührt, und man anschließend die Lösung bis zur Trocknung des Rückstandes, wie oben angegeben, aufarbeitet.

In einer weiteren vorteilhaften Ausführungsform verfährt man wie unter a) und b), setzt Alkalimetallcarbonat bis zu einer Konzentration von 0,35 m zu und arbeitet wie unter d) beschrieben weiter.

Ein weiteres Herstellungsverfahren ist dadurch gekennzeichnet, daß man eine Calciumcarbonat oder -oxidlösung mit einem Alkalimetallcarbonat und Citronensäure in konzentrierter wäßriger Lösung in einem Molverhältnis von Calciumcarbonat, Alkalimetallcarbonat und Citronensäure von 6:3:7 bis 6:3,75:7 bei einem pH-Bereich von 4,6 bis 4,8 bei einer Temperatur von 80-95°C unter Rückfluß während 1 bis 1,5 Stunden unter Rührung umsetzt und man entweder nach der Umsetzung die Lösung mit dem Niederschlag filtriert, den Rückstand mindestens einmal mit Wasser wäscht und trocknet, oder man nach der Umsetzung die Lösung mit dem gebildeten Niederschlag sofort bis zur Trockne einengt, oder daß nach der Umsetzung die Lösung mit dem gebildeten Niederschlag als Suspension das Endprodukt ist.

Dabei zeigt sich, daß es vorteilhaft ist, wenn man
a) einer 1,89 m wäßrigen Citronensäure-Lösung unter Rühren portionsweise Calciumcarbonat in einer solchen Menge zugibt, daß eine Konzentration von 1,62 m Calciumcarbonat vorliegt,
b) unter Wasserzugabe , vorzugsweise 1,5 Stunden lang, rührt,
c) Alkalimetallcarbonat bis zu einer Konzentration von 0,55 m zusetzt,
d) die Lösung , vorzugsweise 0,5 Stunden lang, rührt, und man anschließend die Lösung, wie oben angegeben, einengt.

In einer weiteren vorteilhaften Ausführungsform verfährt man wie unter a) und b), setzt Alkalimetallcarbonat bis zu einer Konzentration von 0,68 m zu und arbeitet wie unter d) beschrieben weiter.

Außerdem zeigt es sich, daß man vorteilhafterweise nach der Waschung der Rückstände eine Säure, z.B. Citronensäure oder HCl, in pharmakologisch vertretbaren Mengen als Stabilisator zugeben kann, um eine Hydrolyse des Endproduktes zu vermeiden. Zur Trocknung der Lösungen oder der gewaschenen Rückstände eignen sich besonders Trockenbleche oder Tellertrockner.

### Herstellungsbeispiel 1

Darstellung von Calcium-Natriumcitrat der Formel: CaNaC₆H₅O₇ 57 kg Calciumcitrat^{.}4H₂0 werden in 180 l Wasser suspendiert. Parallel hierzu werden 29,4 kg Natriumcitrat^{.}2H₂0 und 9,6 kg Citronensäure in 180 l Wasser gelöst. Diese klare Lösung wird unter Rühren zur Suspension gegeben und unter Rühren auf 80°C aufgeheizt sowie unter Rückfluß 1,5 Stunden auf Temperatur gehalten. Während dieser Zeit gibt man portionsweise ein weiteres Mal bis zu 180 l Wasser hinzu. Anschließend wird die Lösung filtriert und der Rückstand mindestens einmal mit Wasser gewaschen. Danach wird der Rückstand auf Trockenblechen getrocknet.

Zur Charakterisierung von CaNaC₆H₅O₇ werden die Röntgenbeugungslinien angegeben, die nach dem Guinier-Verfahren mit CuKα-Strahlung entsprechend dem Beugungswinkel 4θ gemessen wurden.
20.50; 22.20; 23.05; 30.05; 34.40; 43.00; 45.45; 49.80; 58.25; 59.75; 61.30; 63.25; 68.05; 70.03; 71.30; 75.70; 78.80; 81.20; 84.30; 86.90; 89.40; 92.30.

### Herstellungsbeispiel 2

Darstellung einer Mischung, welche i.w. Calcium-Natriumcitrat der Formel: CaNaC₆H₅O₇und Citronensäure enthält.
Die Herstellung erfolgt entsprechend der Vorschrift von Herstellungsbeispiel 1, wobei jedoch anstelle der Filtration der Lösung nach der Umsetzung sich eine sofortige Trocknung der Lösung anschließt. Die Trocknung erfolgt auf Trockenblechen.

Die Gehaltsbestimmung des so erhaltenen Produkts ergab folgende Werte ([%] bezogen auf wasserfreie Substanz)

| | Natrium | Calcium | Citrat |
|---|---|---|---|
| berechnet | 8.09 | 14.10 | 77.63 |
| gefunden | 7.79 | 14.36 | 77.53 |

### Herstellungsbeispiel 3

Darstellung von Calcium-Natriumcitrat der Formel: CaNaC₆H₅O₇ 114,1 kg Calciumcitrat^{.}4H₂0 werden in 360 l Wasser suspendiert. Parallel werden 57,6 kg Citronensäure in etwa 360 l Wasser gelöst und unter Rühren zu der Calciumcitrat-Suspension gegeben. Nach dem Aufheizen wird die Lösung unter Rückfluß auf etwa 80°C für etwa 1,5 Stunden gehalten. Während dieser Zeit werden bis zu 360 l Wasser zugesetzt. Anschließend versetzt man portionsweise mit 31,8 kg wasserfreiem Natriumcarbonat und hält die Temperatur weitere 30 Minuten auf 80°C. Anschließend wird die Lösung filtriert und der Rückstand mindestens einmal mit Wasser gewaschen. Danach wird der Rückstand auf Trockenblechen getrocknet.

### Herstellungsbeispiel 4

Darstellung einer Mischung, welche i.w. Calcium-Natriumcitrat der Formel: CaNaC₆H₅O₇ und Citronensäure enthält.
Die Herstellung erfolgt entsprechend der Vorschrift von Herstellungsbeispiel 3, wobei jedoch statt der Filtration der Lösung und Waschung des Rückstandes die Lösung nach der Umsetzung sofort getrocknet wird. Die Trocknung erfolgt auf Trockenblechen oder auf Tellertrocknern.

### Herstellungsbeispiel 5

Darstellung von Calcium-Natriumcitrat der Formel: CaNaC₆H₅O₇ 134,4 kg Citronensäure werden in 370 l Wasser gelöst. Unter Rühren wird portionsweise 60 kg Calciumcarbonat zugegeben. Die Lösung wird auf 80°C aufgeheizt und unter Rückfluß 1,5 Stunden auf Temperatur gehalten. Während dieser Zeit wird nochmals bis zu 180 l Wasser zugegeben. Dazu erfolgt das portionsweise Zusetzen von 31,8 kg wasserfreiem Natriumcarbonat. Die Lösung wird danach weitere 30 Minuten auf 80°C gehalten. Anschließend wird die Lösung filtriert und der Rückstand mindestens einmal mit Wasser gewaschen. Danach wird der Rückstand auf Trockenblechen getrocknet.

### Herstellungsbeispiel 6

Darstellung einer Mischung, welche i.w. Calcium-Natriumcitrat der Formel: CaNaC₆H₅O₇ und Citronensäure enthält.
Die Herstellung erfolgt entsprechend der Vorschrift von Herstellungsbeispiel 5, wobei jedoch anstelle der Filtration der Lösung und Waschung des Rückstandes sich eine sofortige Trocknung der Lösung anschließt. Die Trocknung erfolgt auf Trockenblechen.

Ein weiterer Gegenstand der Erfindung ist ein pharmazeutisches Mittel, welches dadurch gekennzeichnet ist, daß es ein oder mehrere Calcium-Alkalimetallcitrate der Formel CaXC₆H₅O₇, worin X ein Alkalimetall ist, enthält. Es hat sich gezeigt, daß das erfindungsgemäße Calcium-Alkalimetallcitrat als Wirkstoff in Form eines Arzneimittels, wie in den nachfolgenden Vergleichsbeispielen aufgeführt, besonders geeignet ist. Vorzugsweise kann das Alkalimetall Natrium oder Kalium sein. Weiterhin ist es vorteilhaft, wenn das erfindungsgemäße pharmazeutische Mittel Calcium-Alkalimetallcitrate als Hydrat oder Hydratgemisch enthält. Dabei ist es besonders vorteilhaft, daß das pharmazeutische Mittel einen oder mehrere Zusatzstoffe enthält, wobei sich insbesondere Citronensäure als Zusatzstoff eignet.

Nach einer bevorzugten Ausführungsform der Erfindung kann das Arzneimittel als Tablette, Dragee, Kapsel, Pille, Granulat, Emulsion, Brausetablette oder Lösung appliziert werden, wobei deren Wirkstoffgehalt einem Bruchteil oder einem Vielfachen einer Einzeldosis entspricht. Die Dosierungseinheiten können zum Beispiel 1, 2, 3 oder 4 Einzeldosen oder 1/2, 1/3 oder 1/4 einer Einzeldosis enthalten.

In einer weiteren Ausführungsform können nicht-toxische, inerte pharmazeutisch geeignete Zusatzstoffe als feste, halbfeste oder flüssige Verdünnungsmittel, Füllstoffe und Formulierungshilfsmittel zusammen mit den Wirkstoffen appliziert werden.

Als Zusatzstoffe eignen sich vorzugsweise
(a) Füll- und Streckmittel, zum Beispiel Stärke, Milchzucker, Rohrzucker und Glucose,
(b) Bindemittel, zum Beispiel Gelatine, Carboxymethylcellulose und Alginate,
(c) Feuchthaltemittel, zum Beispiel Glycerin,
(d) Sprengmittel, zum Beispiel Agar-Agar, Calciumcarbonat und Natriumcarbonat,
(e) Lösungsverzögerer, zum Beispiel Paraffin,
(f) Resorptionsbeschleuniger, zum Beispiel quarternäre Ammoniumverbindungen,
(g) Netzmittel, zum Beispiel Glycerinmonostearat,
(h) Adsorptionsmittel, zum Beispiel Kaolin und Bentonit und
(i) Gleitmittel, zum Beispiel Talkum, Calcium- und Magnesiumstearat,
(j) geruchs- oder geschmackverbessernde Zusätze,
(k) Karminativum z.B. Dimeticon
oder Gemische der unter (a) bis (k) aufgeführten Stoffe.

Als Trägerstoff für Lösungen und Emulsionen eignen sich vorzugsweise Wasser, Äthylalkohol und Öle, zum Beispiel Olivenöl, Maiskeimöl und Erdnußöl oder Gemische dieser Stoffe. In einer weiteren Ausführungsform finden sich als Sprengmittel Alkalimetallsalze wie Kaliumhydrogencarbonat oder Natriumhydrogencarbonat und als Hilfsstoff eine physiologische organische Säure. Dabei eignet sich besonders als geschmacksverbessernder Zusatz Saccharin und als Karminativum Dimeticon. Als besonders vorteilhaft erweist es sich, wenn das Arzneimittel als physiologische organische Säure Citronensäure, Weinsäure oder Bernsteinsäure enthält. Die erfindungsgemäße Verwendung erfolgt vorzugsweise oral. Das Arzneimittel kann sowohl als Granulat oder in Form von Brausetabletten problemlos appliziert werden.

### Formulierungsbeispiele

### a) Granulat (Calcium-Natriumcitrat wie auch Calcium-Natriumcitrat mit Citronensäure als Zusatzstoff)

70 kg des nach den Herstellungsbeispielen 1-6 hergestellten Salzes wird zerkleinert, gesiebt und in einen Pelletierteller eingebracht. In den Druckbehälter werden 23 kg gereinigtes Wasser eingewogen. Bei geschlossenem rotierendem Teller mit eingeschaltetem Innenmischer werden die 23 kg Wasser mit einem Druck von 5 bar aufgedüst. Im Anschluß an die Granulierung wird das Granulat auf einem Trockenteller kontinuierlich getrocknet.

### b) Suspension

Zur Herstellung von 20 kg Suspension aus Calcium-Natriumcitrat mit dem Zusatzstoff Citronensäure werden 3,43 kg des nach Herstellungsbeispielen 2, 4 oder 6 hergestellten Salzes unter ständigem Rühren in einer Mischung aus 12,083 kg Lycasin 80/55 und 4,31 kg gereinigtem Wasser suspendiert. Der Ansatz wird unter kräftigem Rühren in Wasserbad auf 70°C erwärmt und 30 Minuten bei dieser Temperatur belassen. Nach dem Erkalten und Ergänzen des verdunsteten Wassers werden 0,167 kg einer 30%igen Lösung vom Natrium-Salz des Methyl-p-hydroxybenzoats in gereinigtem Wasser und 10 g Citronen-Aroma hinzugefügt und nochmals einige Minuten kräftig gerührt. Die fertige Suspension wird direkt in einen fest verschließbaren Behälter gefüllt.

Die Erfindung sei bezüglich der Verwendung von Calcium-Alkalimetallcitraten der Formel CaXC₆H₅O₇ worin X ein Alkalimetall ist oder eines Hydrats oder Hydratgemisches davon zum Aufbau von Muskelproteinen und zur Erhöhung der muskulären Stoffwechselleistung durch antikatabolische Stoffwechselwirkung sowie zur Rekonvaleszenz oder palliativen Behandlung bei kataboler Stoffwechsellage chronisch konsumierender oder degenerativer Krankheiten, vorzugsweise bei Tumorkachexie, Niereninsuffizienz, seniler Kachexie, Alkoholabusus und Infekten, auch bei Strahlen- und Zytostatikatherapie, verlängerter Kortikoidtherapie, postoperativen oder posttraumatischen Zuständen - wobei durch die antikatabolische Wirkung ein Proteinaufbau erfolgt und eine Reduzierung des Allgemeinzustandes des Organismus verhindert wird - in nachfolgenden Studien erläutert:

### A. Vergleichsversuche

Die Vergleichsversuche wurden mit Ratten (unter Beachtung des deutschen Tierschutzgesetzes) durchgeführt, da im Falle von Vergleichsversuchen an Menschen die Versuchsgruppen mit Versuchspersonen mit konsumierenden Erkrankungen in zeitlich identischem Erkrankungsstadium nötig gewesen wären.

Daher wurden Tiermodelle herangezogen, bei denen ein Krankheitsbild simuliert wurde, wie Reduktion des Allgemeinzustandes, z.B. Körpergewichtsabnahme, die auch aufgrund von kataboler Stoffwechsellage durch Proteinabbau hervorgerufen wurde. Zur Überprüfung, ob das zu untersuchende Mittel eine antikatabolische Wirkung, d.h. beispielsweise einen körpergewichtsaufbauenden und somit auch proteinaufbauenden Effekt manifestiert, wurden Ratten kastriert und mit Calcium-Natriumcitrat behandelt.

### a) Methodik:

Männliche, Pentobarbital betäubte Ratten (Sprague-Dawley Ratten) wurden kastriert, wobei die Versuchsgruppen aus mindestens 4 Tieren bestanden.

Folgende Versuchsgruppen wurden untersucht:
**I** Kontrolle: Ratten, deren Bauchhöhle geöffnet und geschlossen wurde, jedoch ohne Kastration und deren Trinkflüssigkeit aus deionisiertem Wasser bestand
**II** Ratten, deren Bauchhöhle geöffnet und geschlossen wurde, mit Kastration und deren Trinkflüssigkeit aus deionisiertem Wasser bestand
**III** Ratten, deren Bauchhöhle geöffnet und geschlossen wurde, mit Kastration, deren Trinkflüssigkeit 0,25 Gew.% einer Mischung mit CaNaC₆H₅O₇ und Citronensäure enthielt.

Vor dem letzten Versuchstag wurden die Tiere 12 h nüchtern gesetzt, dabei die Tränke belassen. Die Blutentnahme erfolgte in Narkose (siehe oben). Zuerst wurde die Schwanzspitze amputiert, um arterio-venöses Mischblut für die sofortige Hämatokrit- und Blutgasbestimmungen zu gewinnen. Anschließend wurde laparotomiert, die abdominale Aorta freigelegt, punktiert, das Tier vollständig entblutet und das Blut in vorgekühlten Röhren gesammelt. Serum-Aliquots für Bestimmungen wurden bei -30°C gelagert. Die Nieren wurden entnommen und nach Steinen durchsucht.

Alle Ergebnisse wurden als arithmetischer Mittelwert ± 1 mittlerer Fehler des Mittelwertes (SEM) angegeben.

### b) Ergebnisse:

Die Tab.1 zeigt eine deutliche Abnahme des Körpergewichtes der Ratten der Gruppe II gegenüber denen der Kontrolle (Gruppe I) um mehr 17 % . Die Abnahme demonstriert, daß die Kastration zumindest bez. des Körpergewichtes und damit auch bez. der Proteinmasse eine katabole Stoffwechsellage wiederspiegelt. Somit ist dieses Tierversuchsmodell in Hinsicht auf Untersuchungen über die Proteindegradation mit dem katabolen Stoffwechsel vergleichbar und heranzuziehen. In Gegenwart von Calcium-Natriumcitrat wird das Körpergewicht im Vergleich zu der Gruppe II erhöht und erreicht annähernd das Körpergewicht der Kontrolle (Gruppe I). Daher ist es zwar richtig, daß keine Unterschiede zwischen der Kontrolle (Gruppe I) und Gruppe III (mit Calcium-Natriumcitrat) nach Verwendung von Calcium-Natriumcitrat zu erkennen sind, jedoch ist es ebenso offensichtlich, daß die Applikation von Calcium-Natriumcitrat auf die durch die Kastration hervorgerufene Proteinolyse (Gruppe II) verstärkt antikatabolisch, also anabolisch, wirkt und die Proteinbiosyntheserate stark erhöht. Folglich führt die antikatabolische Wirkung der verabreichten Verbindung zu einer Positivierung der Stickstoffbilanz.

Das bedeutet, daß Calcium-Natriumcitrat zu einer Erhöhung der Proteinmasse im Körper und somit zur Erhöhung der Muskelmasse beiträgt. Weiterhin ist festzustellen, daß die Einnahme von Calcium-Natriumcitrat auf die Nierentätigkeit, insbesondere auf die Flüssigkeitsresorption der Nieren vernachlässigbar gering ist, sodaß, da bezüglich des Urinvolumens und des Flüssigkeitsverbrauchs zwischen den drei Gruppen I, II und III kein relevanter Unterschied besteht, auch zu sehen ist, daß eine möglicherweise längere Einnahme von Calcium-Natriumcitrat ebenso keine Veränderungen im Flüssigkeitshaushalt des Organismus hervorrufen wird.

Auch bei der Darmausscheidung und der partiellen Absorption von Calcium, Magnesium und Phosphor sind keine Unterschiede erkennbar, so daß gleichfalls in Hinsicht auf diese Parameter mit Nebenwirkungen im Bereich der Nahrungsverarbeitung im Darm nicht zu rechnen ist und eine komplikationslose Verstoffwechslung erfolgt.

Bemerkenswert war, daß überraschenderweise die Futteraufnahme der Gruppe III verringert war, jedoch die Nahrungseffizienz, also die Verstoffwechslung der Nahrung, im Vergleich zu denen der Gruppen I und II stark erhöht war und um ca. 80 bis 100 % über den Vergleichswerten der Gruppen I und II lag, so daß hierbei die antikatabolische Wirkung von Calcium-Natriumcitrat besonders gut demonstriert wurde.

**Tab. 1:**

| **Allgemeiner Status der Versuchstiere** | | | |
|---|---|---|---|
| | I n = 4 | II n = 4 | III n = 4 |
| Körpergewicht | | | |
| anfangs | 110 ± 3 | 108 ± 12 | 112 ± 3**** |
| zuletzt | 441 ± 11 | 365 ± 10*** | 423 ± 22 |
| Unterschied | 301 ± 10 | 257 ± 12*** | 301 ± 20 |
| | | | |
| Flüssigkeit* [ml/Tag/100g KG] | 9,6 ± 0,9 | 9,0 ± 0,6 | 9,5 ± 1,9 |
| | | | |
| Futteraufnahme [g/Tag/100 g KG] | 6,0 ± 0,6 | 6,0 ± 0,3 | 3,4 ± 0,2*** |
| | | | |
| Futterverwertung** [g/g] | 11,8 ± 1,4 | 10,8 ± 0,4 | 20,6 ± 1,3*** |
| | | | |
| Urin* [ml/Tag/100 g KG] | 8,3 ± 1,3 | 5,5 ± 0,4 | 6,7 ± 2,0 |

| | | | |
|---|---|---|---|
| Mittelwerte +/- SEM. * basierend auf den Mittelwerten von drei aufeinanderfolgenden Tagen, an welchen die Tiere in Stoffwechselkäfigen untergebracht waren. | | | |
| ** Körpergewichtszunahme, in g (siehe *) dividiert durch Nahrungsaufnahme (siehe *), in g. | | | |
| *** p<0.01 versus Kontrolle (Gruppe I) (Bonferroni-Prinzip berücksichtigt) | | | |
| **** p<0.025, KG: Körpergewicht n Rattenanzahl | | | |

Der Umstand, daß Calcium-Alkalimetallcitrate ohne Nebenwirkungen im Gegensatz zu den bekannten o.g. Medikamenten auf die Nierentätigkeit sind, ergab sich auch aus den Werten für Hämatokrit und für Serumkreatinin, welche im normalen Bereich blieben, was auf einen hinsichtlich Osmolalität unveränderten extravasalen Raum bzw. eine normale Nierenfunktion in allen Gruppen hinwies, insbesondere in der mit Calcium-Natriumcitrat behandelten Gruppe III (Daten nicht angegeben).

Aufgrund der Bedeutung der Mineralien bei den vorliegenden Experimenten, wird die Elektrolyt-Bilanz in Tab. 2 dargestellt. Die Calciummenge im Serum blieb jedoch in allen Gruppen konstant. Unbedenklich und gleichbleibend, verglichen mit Gruppe I, waren gleichfalls die Serum-Werte für Phosphor und Osteocalcin. Ebenso konstant waren die im Urin festgestellten Konzentrationen an Phosphor, Magnesium und cyclischem AMP im Vergleich zur Kontrolle. Diese Ergebnisse weisen somit eindrucksvoll aufgrund der gleichbleibenden Konzentrationen der untersuchten Mineralien und Ionen im Serum und im Urin auf die vernachlässigbaren Wirkungen von Calcium-Alkalimetallcitraten auf den Elektrolyt-Haushalt im Blut als auch auf die Nierentätigkeit hin.

**Tab. 2:**

| **Daten zu Serum und Urin (Mittelwerte +/- SE) Zu weiteren Einzelheiten siehe Angaben in Tab.1.** | | | |
|---|---|---|---|
| | I n=4 | II n=4 | III n=4 |
| Serum | | | |
| Calcium [mg/dl] | 9,3 ± 0,1 | 9,4 ± 0,4 | 10,2 ± 0,5 |
| Magnesium [mg/dl] | 2,3 ± 0,1 | 2,5 ± 0,0**** | 3,1 ± 0,8 |
| Phosphor [mg/dl] | 8,8 ± 0,4 | 8,4 ± 0,5 | 10,5 ± 2,1 |
| Osteocalcin [ng/ml] | 53 ± 6 | 56 ± 4 | 49 ± 5 |

| Urin | | | |
|---|---|---|---|
| Citrat/Cr# [mmol/mmol] | 0,78 ± 0,05 | 1,11 ± 0,09*** | 1,03 ± 0,19 |
| Magnesium/Cr [mmol/mmol] | 2,2 ± 1,5 | 1,5 ± 0,6 | 2,2 ± 0,7 |
| Phosphor/Cr [mmol/mmol] | 4,5 ± 0,6 | 6,3 ± 0,3*** | 5,1 ± 1,0 |
| cAMP/Cr [µmol/mmol] | 1,7 ± 0,3 | 1,5 ± 0,4 | 1,8 ± 0,3 |

| | | | |
|---|---|---|---|
| #: Kreatinin in derselben Harnprobe | | | |
| *** p< 0,01 versus Kontrolle (Gruppe I) (Bonferroni-Prinzip berücksichtigt) | | | |
| **** p< 0,025 | | | |

Überraschenderweise sind die Citrat-Werte im Urin bei der Gruppe III nur unbedeutend höher, trotz der Tatsache, daß mit Calcium-Natriumcitrat eine zusätzliche Citrat-Menge verabreicht wird, was als Zeichen für eine schnelle Verteilung und Verstoffwechslung der Verbindungen spricht.

Darüberhinaus lag der Blut-pH-Wert bei allen Versuchsgruppen im konstanten Bereich (Tab. 3). Auch diese Daten verdeutlichen, daß trotz der Einnahme von Calcium-Natriumcitrat der Einfluß auf das Säuren-Basen-Milieu im Blut erkennbar gering und somit vernachlässigbar war.

**Tab. 3**

| **Blut-pH** | | | |
|---|---|---|---|
| Gruppen | I | II | III |
| pH | 7.38 ± 0.01 | 7.34 ± 0.01 | 7.36 ± 0.03 |

Diese Versuche zeigen somit eindrucksvoll und in einer für den Fachmann nicht vorhersehbaren Eindringlichkeit, daß überraschenderweise die Applikationen mit Calcium-Alkalimetallcitraten der o.g. Formel aufgrund der gleichbleibenden Konzentrationen der Stoffwechselprodukte sowohl im Harn als auch im Blut zu einer ausgezeichneten Verträglichkeit der Substanzen im Organismus als auch zu keinen nennenswerten Nebenwirkungen führen und diese Verbindungen aufgrund ihrer antikatabolischen Wirkungen einen verstärkten Proteinaufbau hervorrufen.

Die Untersuchungen demonstrieren, daß Calcium-Natriumcitrat weder die Nierentätigkeit aufgrund der Konstanz in den Serumanalysewerten und im mineralischen Stoffwechselhaushalt beeinflußt noch in den Flüssigkeitshaushalt störend eingreift. Calcium-Alkalimetallcitrate der o.g. Formel sind somit gut und schnell im Organismus abbaubar. Da Calcium-Alkalimetallcitrate im Organismus zu Citrat dissoziieren, sollten sie auch in Kombination mit weiteren Wirkstoffen, solange diese nicht mit dem körpereigenen Citrat in Wechselwirkung treten, zu keinen Kontraindikationen führen. Auch sind keine androgenen Nebenwirkungen dieser Verbindungen aufgrund ihrer vollkommen andersartigen Struktur zu erwarten.

Da zur Therapie ein Derivat einer körpereigenen Substanz, i.e. Citrat-, Calciumverbindungen bzw. deren Salze, appliziert wird, und da in den Vergleichsversuchen bei der Einnahme von Calcium-Natriumcitrat keine Änderungen im Stoffwechselhaushalt zu erkennen waren, ist ebenso eine Langzeiteinnahme möglich, wie es gerade sowohl die Einnahmen von Calcium-Alkalimetallcitraten der o.g. Formel durch Leistungssportler zum Muskelproteinaufbau und zur Erhöhung der Stoffwechselleistung als auch die frühzeitig beginnenden Verabreichungen mit Calcium-Alkalimetallcitraten der o.g. Formel zur Rekonvaleszenz oder palliativen Behandlung von konsumierenden Erkrankungen erforderlich machen.

Außerdem wies der zurückgegangene Futterverbrauch und der verminderte Kreatiningehalt im Urin in Gegenwart von Calcium-Natriumcitrat auf eine geringe Umwandlung von Muskelproteinen hin. Das bedeutet, daß die negative Beziehung von Futtereffizienz und Kreatiningehalt im Urin eine antikatabolische Stoffwechselphysiologie im Organismus widerspiegelte.

### B. Klinische Prüfung:

Patienten wurde nach akuter Colitis ulcera während der Rekonvaleszenz (Erholungsphase) Calcium-Natriumcitrat verabreicht. Wegen des außerordentlichen hohen methodischen Aufwandes, der hauptsächlich darin bestand, daß bei diesen Patienten eine Vielzahl von Parametern ständig zu beobachten war, und wegen der zu erwartenden hohen Aussagefähigkeit von Veränderungen des Stoffwechsels, wurde die Patientenanzahl möglichst gering gehalten. Es zeigte sich dabei, daß in den Versuchen jeweils drei Patienten vollkommen ausreichend waren.

### a) Methodik:

Diese Patienten wurden kontrolliert ernährt und es wurde ihnen über eine Duodenalsonde Nahrung zugeführt, die in ihrer Menge und Zusammensetzung genauestens bekannt war. Die Patienten waren ferner vollbilanziert, d. h. Nahrungszufuhr- und -ausscheidung sowie ein breites Spektrum von Stoffwechselgrößen, darunter der Umsatz von Proteinen, Fett und Kohlenhydraten wurden ständig gemessen. Im Versuchsablauf wurden die Patienten über 3 Tage mit 2 x 5 g Calcium-Natriumcitrat behandelt und vor, während und nach dieser Phase gemessen.

### b) Ergebnisse

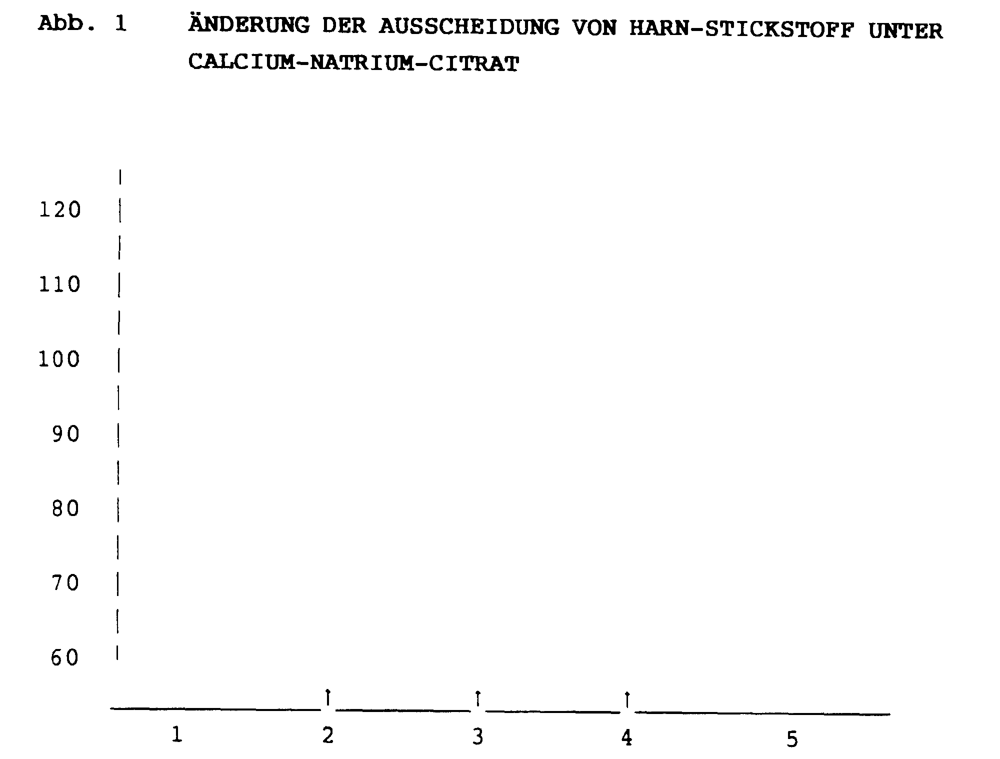

Änderung der Ausscheidung von Harn-Stickstoff anhand der exemplarischen Wiedergabe der Werte eines Patienten in Gegenwart von Calcium-Natriumcitrat
Ordinate: Relative Änderung der Harn-N-Ausscheidung (%)
Abszisse: Versuchsdauer (Tage), der Pfeil gibt die Zeitpunkte der Applikation mit Calcium-Natriumcitrat (2 x 5 g/Tag) wieder 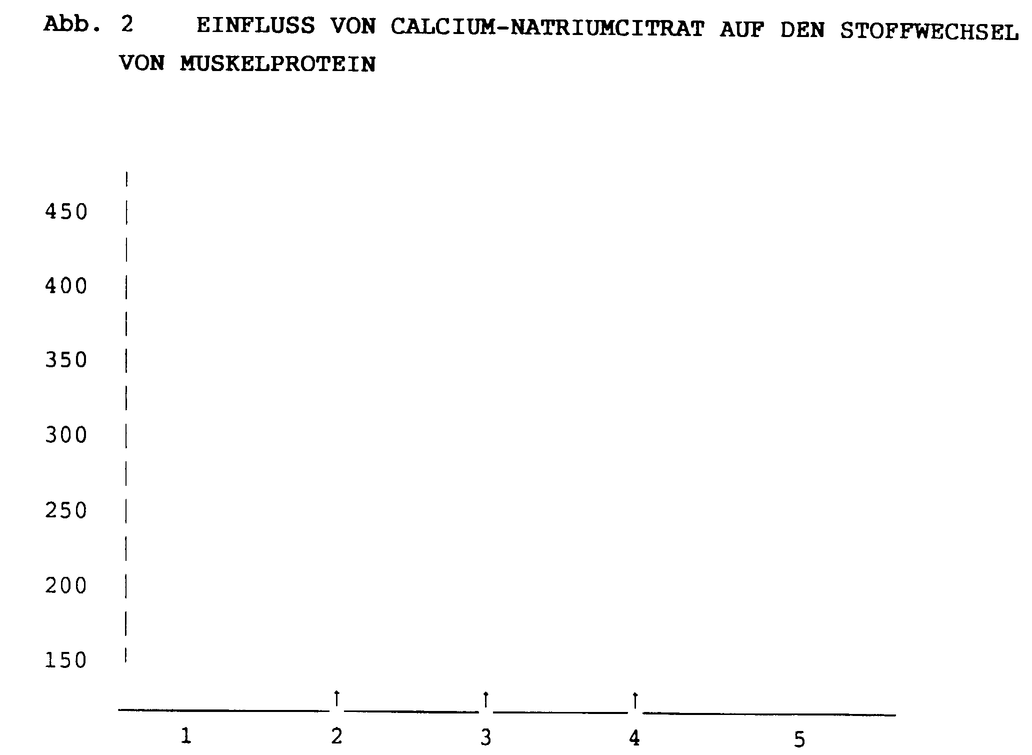 Einfluß von Calcium-Natriumcitrat auf den Stoffwechsel von Muskelprotein
Ordinate: 3-Methyl-Histidin (µmol/Tag)
Abszisse: Versuchsdauer (Tage), der Pfeil gibt die Applikationen mit Calcium-Natriumcitrat (2 x 5 g/Tag) wieder

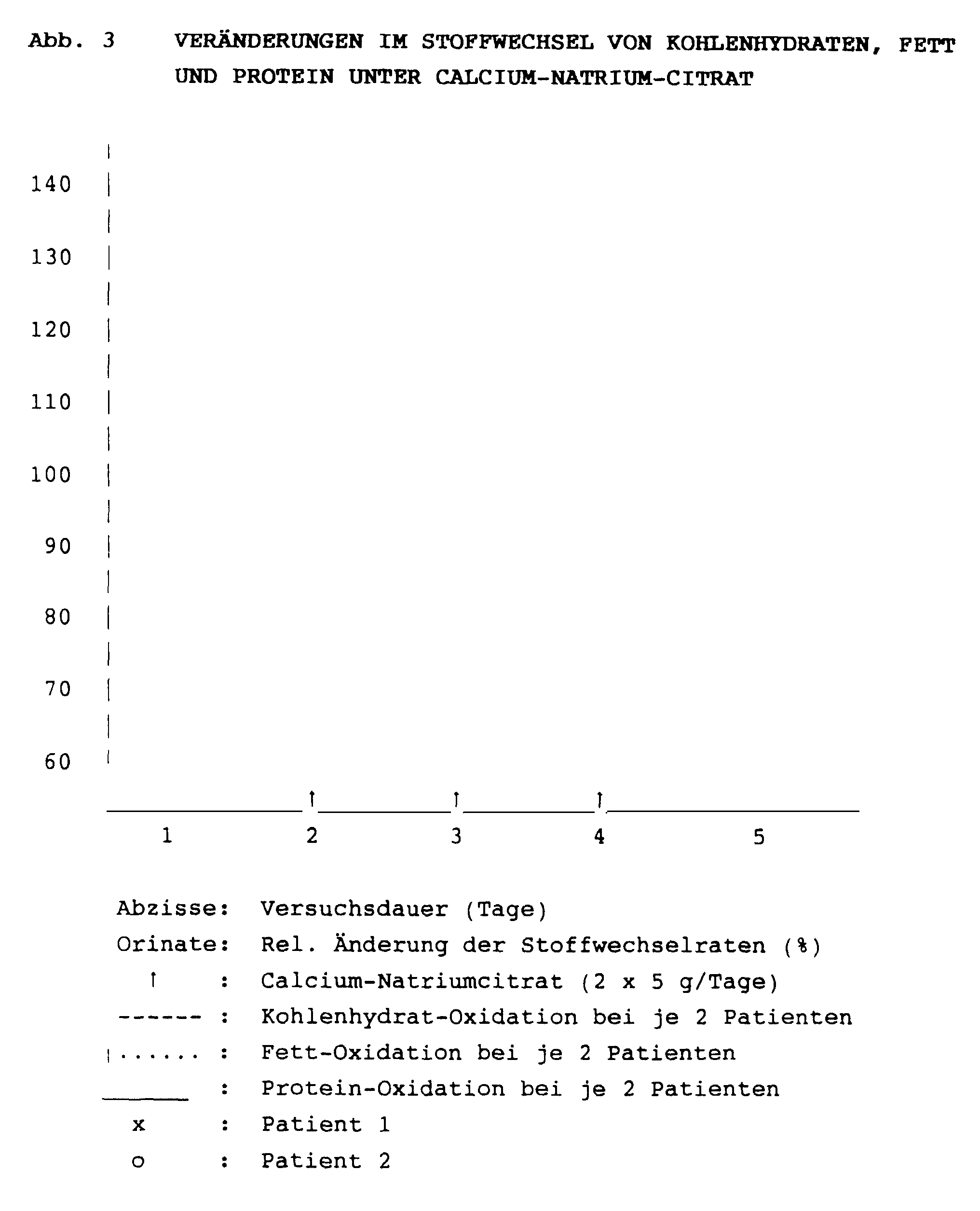

Abb. 1 zeigt die Ausscheidung von Stickstoff im Harn. In Gegenwart von Calcium-Natriumcitrat nimmt der Harn-Stickstoff in der Größenordnung von 20-30% ab, die sehr beachtlich ist. Da Proteinabbauprodukte nahezu ausschließlich als Harnstoff im Urin ausgeschieden werden, weist diese eindrucksvolle Abnahme von Stickstoff auf eine Proteineinsparung hin. Der katabole Stoffwechsel wird in Hinsicht auf die Proteindegradation im Organismus und somit auch in Hinsicht auf die Muskelproteinolyse durch die Applikation von Calcium-Natriumcitrat aufgrund der antikatabolischen Wirkung inhibiert.

Bei einem Patienten wurde in Kenntnis des Proteineinspareffektes auch die Ausscheidung von 3-Methylhistidin im Urin gemessen (Abb.2). Dieses Aminosäure-Derivat ist ein aussagefähiger Indikator des Proteinstoffwechsels in der Muskulatur. Man sieht, daß die Ausscheidung von 3-Methylhistidin unter dem Einfluß von Calcium-Natriumcitrat stark zurückgeht. Dies bedeutet, daß der Verbrauch an Muskelproteinen unter dieser Medikation rückläufig ist, somit vor allem Muskeleiweiß eingespart wird.

Abb. 3 zeigt den Verbrauch von Fett, Eiweiß und Kohlenhydraten anhand der jeweiligen Oxidationsrate vor und nach der Calcium-Natriumcitrat-Zugabe. Der Proteineinspareffekt an Körpereiweiß ist eindeutig zu erkennen. In Hinsicht auf den Fettstoffwechsel gibt es nur leichte Einspareffekte. Die Rate der Kohlenhydratoxidation in Gegenwart von Calcium-Natriumcitrat steigt stark an. Zusammen mit dem Proteineinspareffekt ist eine eindeutige Zunahme der Kohlenhydratoxidation unter Calcium-Natriumcitrat zu verzeichnen.

Diese Ergebnisse erhärten somit die bereits mit dem Tiermodell ermittelten Resultate, daß überraschenderweise Calcium-Natriumcitrat der o.g. allgemeinen Formel antikatabolisch im menschlichen Organismus wirkt, die Proteinolyse hemmt, die Proteinbiosynthese und die Kohlenhydratoxidation steigert, ohne den Nierenstoffwechsel sowie den Elektrolythaushalt nennenswert zu beeinflussen und ohne daß androgene Nebenwirkungen zu erwarten sind.

### C. Vergleichsversuche mit Sportlern

Verabreicht wurde eine 20% Suspension mit 2 g Calcium-Natriumcitrat auf 10 g Endpräparat mit Zitronengeschmack und 1 g Glukose auf 10 g, welche in Aluminiumbeuteln verpackt waren.
a) Leistungsportlern im Schwimmen als Versuchspersonen wurde 16 g der Formulierung, auf drei Stunden verteilt, vor Beginn der Übung ohne Auftreten von Unverträglichkeiten verabreicht. Dabei zeigt sich nach der Applikation sowohl bei 100m als auch 400m Leistungsmodalität eine Steigerung um ca. 2 bis 4% im Hochleistungsbereich.

**Tab.: 4**

| **Steigerung der Stoffwechselleistung bei Sportlern ermittelte Zeiten** | | |
|---|---|---|
| Disziplin | ohne | mit Calcium-Natriumcitrat |
| 100 m | 52'' 51 | 50'' 75 |
| | 56'' 52 | 55'' 00 |
| 400 m | 4' 25'' 52 | 4' 20'' 78 |
| | 3' 52'' 06 | 3' 47'' 43 |

Nach Aussagen der Sportler fühlten sie sich auch stärker belastbar als ohne die Einnahme von Calcium-Natriumcitrat.
b) Langstreckenläufer als Versuchspersonen unterzogen sich einem Belastungstest auf einem Laufband, wobei der Versuch bei 10 km/h begann und bei einer maximalen Geschwindigkeit von 20 km/h die allgemeine Belastungsgrenze erreicht wurde. Der Test wurde von 8 Versuchspersonen mit und ohne Calcium-Natriumcitrat durchgeführt.
Gemessen wurden die biochemischen Parameter Blut-pH, pO₂, CO₂ insgesamt, Laktat und kardio-respiratorische Parameter: EKG, Blutdruck, Herzfrequenz, VO₂max, VCO₂, RQ und FO₂. Blutproben wurden nach 0 , 5, 10 und 15 min entnommen.
Die Ergebnisse zeigen eindeutig eine Verzögerung der Ermüdungserscheinungen und eine Verkürzung der Erholungsphasen, wie exemplarisch anhand der Blut-pH-Werte in Tab. 5 demonstriert wird. Der Blut-pH wird innerhalb des alkalischen Bereichs aufrechterhalten, sodaß eine Erholung schneller ermöglicht wird. Ebenso fühlen sich die Sportler einhellig belastbarer als ohne Calcium-Natriumcitrat-Zugabe. Die Tab. 5 zeigt, daß über einen langen Zeitraum der Blut-pH alkalisch bleibt trotz der hohen Leistungsanforderungen an die Sportler.

**Tab.: 5**

| **Blut-pH-Werte bei Belastung nach 0, 5, 10 und 15 Min.** | | | | | |
|---|---|---|---|---|---|
| Proband | | **0** | **5** | **10** | **15** |
| 1 | a | 7,32 | 7,23 | 7,26 | 7,29 |
| | b | 7,24 | 7,14 | 7,17 | 7,18 |
| | | | | | |
| 2 | a | 7,39 | 7,35 | 7,35 | 7,36 |
| | b | 7,35 | 7,20 | 7,26 | 7,29 |
| | | | | | |
| 3 | a | 7,38 | 7,21 | 7,23 | 7,27 |
| | b | 7,33 | 7,08 | 7,11 | 7,14 |
| | | | | | |
| 4 | a | 7,36 | 7,24 | 7,26 | 7,30 |
| | b | 7,37 | 7,24 | 7,21 | 7,21 |

| | | | | | |
|---|---|---|---|---|---|
| a: mit Calcium-Natriumcitrat | | | | | |
| b: ohne Calcium-Natriumcitrat | | | | | |

c) Studie über Dosis/Wirkung bei Übungen unter aeroben Bedingungen
Die Studie wurde mit 10 gesunden und geübten Sportlern durchgeführt, die wöchentlich zwischen 10 und 15 Stunden Sport unter aeroben Bedingungen betreiben und deren Alter zwischen 18 und 40 Jahren lag. Für den Versuch wurde ein Zykloergometer und ein Pulsometer für die Messung der Herzfrequenz verwendet, speicherprogrammiert und die Werte alle 5 Sekunden aufgezeichnet. Für die Bewertung der sportlichen Leistung wurde der zykloergometrische Test PWC-170 ausgewählt, aufgrund der großen Erfahrung mit besagtem Test und weil dieser von der Europäischen Gemeinschaft anerkannt ist. Das verwendete Protokoll zeichnete die Herzfrequenz bei 50W, 100W, 150W und 250W Belastung auf. Diese Belastung wurde nach und nach im Abstand von 3 Minuten erhöht, bis der Proband 170 Pulsschläge erreichte. Zu diesem Zeitpunkt wurde der Versuch abgesetzt und der Puls wurde 1, 3 und 5 Minuten nach der Anstrengung gemessen. Berechnet wurde der VO₂/ml/kg/Minute bei den erzielten Daten.

Parallel dazu wurden Messungen des Harn-pH-Werts mit Digitalmesser im Basal-Zustand vorgenommen, eine und 2 Stunden nach Einnahme von Calcium-Natriumcitrat. In der Studie wurden anfänglich 10 Versuche unter Beanspruchung in vorgenannter Weise durchgeführt, 1 Stunde zuvor wurden 6 g Calcium-Natriumcitrat verabreicht. Die Versuche wurden nach 48 Stunden wiederholt, ohne irgend etwas einzunehmen, es wurden gleiche Parameter verzeichnet unter identischen Bedingungen hinsichtlich Temperatur, Feuchtigkeit und Luftdruck. Unter den 10 Personen, die den ersten Versuch durchführten, wurden 5 für einen zweiten Versuch unter den gleichen Besonderheiten, aber mit einer 10 g Calcium-Natrium-Citrat-Dosis per Zufall ausgewählt.

Die Ergebnisse zeigen, daß weder Nebenwirkungen noch Unverträglichkeit hinsichtlich der verabreichten Dosen von Calcium-Natriumcitrat festgestellt werden. Es werden auch keine relevanten Verbesserungen der Parameter Herzfrequenz, Sauerstoffverbrauch oder Rekuperation nach Anstrengung bei den Probanden beobachtet, die den Versuch mit 6 g der Formulierung ausführten, im Vergleich zu jenen, die ohne Citrat (Kontrolle) teilnahmen.

Bei der Versuchsgruppe, die später mit 10 g der Formulierung eingesetzt wurden, stellte man jedoch bedeutsame Unterschiede hinsichtlich der Pulsschläge, des Sauerstoffverbrauchs und der Erholung nach der Anstrengung fest. Die Studie des Harn-pH-Werts zeigte eine Alkalisierung des Harns eine Stunde nach der Applikation, sowohl bei einer Dosis von 6 als auch bei 10 g, wobei besagte Alkalisierung bei den Personen bis zu 2 Stunden fortbestand, die 10 g genommen hatten, mit 2 Ausnahmen, die ein höheres Gewicht im Verhältnis zur Körpergröße aufwiesen und bei denen die Alkalisierung nur 1 Stunde anhielt. Das traf auch bei den Personen zu, die 6 g der Formulierung eingenommen hatten.

**Tab.: 6**

| **Dosis/Wirkung-Studie** | | | |
|---|---|---|---|
| | Formulierung | | Kontrolle |
| Zeitpunkt | 6 g | 10g | 0g |
| pH Basal | 5,52 | 5,57 | 5,62 |
| 60 min | 6,78 | 6,00 | 6,30 |
| 120 min | 5,88 | 7,15 | 5,67 |

Aus den durchgeführten Versuchen kann abgeleitet werden, daß die Alkalisierung und der durch Calcium-Natriumcitrat erzielte Puffereffekt die Stoffwechselleistung sowie die Erholung nach Anstrengungen unter aeroben Bedingungen verbessern. Die Herabsetzung der Herzfrequenz bei identischer Belastung, die Verbesserung des Sauerstoffverbrauchs und die Verkürzung der Erholungszeit, die mit einer Dosis von 10 g der Formulierung erreicht wurden, sind ein eindeutiger Beweis für den Vorteil von Calcium-Natriumcitrat in der Sportmedizin.

Die auch kurzfristig mögliche, größere Leistungsbreite, wie weniger Müdigkeitsgefühl, kürzere Erholungsphasen und starke Abpufferung vom pH im Blut zusammen mit dem gleichzeitigen Bereitstellen von zusätzlichen Proteinreserven sowie der erhöhten Kohlenhydratoxidation im Organismus durch die antikatabolischen Wirkungen von Calcium-Natriumcitrat bei längerer Einnahme des Wirkstoffes ermöglicht überraschenderweise die Stoffwechselleistung gerade bei Leistungssportlern eindrucksvoll zu erhöhen, ohne daß die bei der Applikation von Hormonen, wie Androgenen, typischerweise auftretenden virilisierenden Effekte und die weiteren o.g. Nebenwirkungen zu diagnostizieren sind.

Die Erfindung sei bezüglich der Verwendung von Calcium-Alkalimetallcitraten der Formel CaXC₆H₅O₇ worin X ein Alkalimetall ist oder eines Hydrats oder Hydratgemisches davon, zur Prophylaxe oder Behandlung des Knochengewebeschwundes und zur Verstärkung der Knochenstrukturelemente sowie zur Prophylaxe oder Behandlung der Osteoporose in nachfolgenden Studien erläutert:

### Vergleichsversuche

Es wurden Tiermodelle herangezogen, insbesondere Tiere mit kurzer Lebens spanne und einem Knochenstoffwechsel mit hohem Umsatz. Die Vergleichsversuche mit Ratten entsprechen im Ergebnis denen bei Menschen bez. der Identität des Abbaus von Knochensubstanz und der Physiologie der Erkrankung, da für das Studium mineral-metabolischer Komplikationen wie Osteoporose oder Osteopenie infolge Hypogonadismus der Zustand nach Orchiektomie als geeignete Situation betrachtet wird (Stepan et al., J.Clin. Endocr. Metab. 69, 523 - 527, 1989).

### a) Methodik:

Männliche, Pentobarbital betäubte Ratten (Sprague-Dawley Ratten) wurden kastriert, wobei die Versuchsgruppen aus mindestens 4 Tieren bestanden.

Folgende Versuchsgruppen wurden untersucht:
- **SHAM**: Kontrolle: Ratten, deren Bauchhöhle geöffnet und geschlossen wurde, jedoch ohne Kastration, deren Trinkflüssigkeit aus deionisiertem Wasser bestand
- **OR-X**: Ratten, deren Bauchhöhle geöffnet und geschlossen wurde, mit Kastration, deren Trinkflüssigkeit aus deionisiertem Wasser bestand
- **OR-X+CNC**: Ratten, deren Bauchhöhle geöffnet und geschlossen wurde, mit Kastration, deren Trink-flüssigkeit 0,25 Gew.% einer Mischung mit CaNaC₆H₅O₇ und Citronensäure (=**CNC**) enthielt

### Markierung des Knochens mit ⁴⁵Calcium:

15 Tage vor Beendigung des Versuchs wurde allen Tieren in Narkose (siehe oben) ⁴⁵Calcium (Amersham, Braunschweig; BRD) unter die Bauchhaut injiziert. Im allgemeinen wurde mit der einmaligen Verabreichung dieser hohen Dosis ⁴⁵Calcium der Pool des rasch austauschbaren Knochencalciums innerhalb dieses Zeitraumes markiert. Unter der Annahme, daß nach dieser Zeit der im Blut zirkulierende Tracer ossärer Herkunft war, konnte außerdem ⁴⁵Calcium in den Faeces als Indikator der endogenen Calcium-Sekretion betrachtet werden. Mit Hilfe dieser Technik wurden die Einflüsse von CNC auf die Bewegungen von Calcium besser transparent gemacht.

Vor dem letzten Versuchstag wurden die Tiere 12 h nüchtern gesetzt, dabei die Tränke belassen. Die Blutentnahme erfolgte in Narkose (siehe oben). Zuerst wurde die Schwanzspitze amputiert, um arterio-venöses Mischblut für die sofortige Hämatokrit- und Blutgasbestimmung zu gewinnen. Anschließend wurde laparotomiert, die abdominale Aorta freigelegt, punktiert, das Tier vollständig entblutet und das Blut in vorgekühlten Röhren gesammelt. Im Serum wurde am selben Tag die Radioaktivität von ⁴⁵Calcium gemessen; weitere Aliquots für andere Bestimmungen wurden bei -30°C gelagert. Die Nieren wurden entnommen und nach Steinen durchsucht. Beide Femora und die linke Tibia wurden ausgehülst und entfettet. Der linke Femur wurde gewogen und das Volumen bestimmt. Anschließend wurde der Knochen getrocknet, gewogen und verascht. Die Asche wurde gewogen, in 10 ml 6 M HCl aufgelöst und die Radioaktivität von ⁴⁵Calcium gemessen. Eine konventionelle Röntgenuntersuchung als Autoradiogramm erfolgte mit dem rechten Femur jeden Tieres.

### Bestimmung des Mineralsalzgehalts mittels Röntgen-Computertomographie:

Da die Beurteilung der Knochendichte auf konventionellen Röntgenaufnahmen schwankt, d.h. nur qualitative Aussagen gestattet, wurde die quantitative Computertomographie (QCT) des rechten Femurs und der linken Tibia angewandt.

Alle Ergebnisse wurden als arithmetischer Mittelwert +/- 1 mittlerer Fehler des Mittelwertes (SEM) angegeben.

### b) Histologische Ergebnisse:

Auf den Röntgenfilmen zeigen eindrucksvoll die Femora von OR-X-Ratten ohne CNC-Zugabe eine deutlich stärkere Durchlässigkeit für Röntgenstrahlen als die der beiden anderen Gruppen (Abb. 4). Das bedeutet, daß ein Knochengewebeschwund in der Versuchsgruppe OR-X zu verzeichnen ist und somit mit der Osteoporose aufgrund des identischen Erscheinungsbildes vergleichbar ist. Durch Zugabe von CNC jedoch wird überraschenderweise der eintretende Knochengewebeschwund aufgehalten und sogar, was noch wichtiger ist in Hinsicht auf bereits einge tretene Osteoporose, die noch verbliebenen Verstärkungselemente angeregt, Knochensubstanz neu zu bilden, da in den auf der Abb. 4c. unteren Bereichen des Femurs der OR-X-CNC Gruppe ein ausgeprägter Knochenaufbau zu erkennen ist. Diese Verstärkungswirkung übertrifft sogar die bereits im Femur der Kontrolle vorhandenen Knocheneinlagerungen Abb. 4a. (SHAM-Gruppe). Auch die indirekte Feststellung des Calciumgehalts im Femur mittels Computertomographie spiegeln die Wirkung von CNC-Zugabe wider (Abb. 5) .

**Abb. 4** Konventionelles Röntgenbild des skelettierten Femurs
a. einer Kontrollratte (SHAM)
b. kastrierten Ratte (OR-X) ohne CNC-Zugabe
c. kastrierten Ratte (OR-X+CNC) mit CNC-Zugabe CNC = Calcium-Natriumcitrat

**Abb. 5** Calciumgehalt des Femurs, bestimmt:
- indirekt mittels Computertomographie (HAP)
- direkt mittels Knochenaschenanalyse (Calcium)
- Knochendichte (Dichte), Knochenaschegewicht (Asche) alle pro Einheit
- Knochenvolumen (offene bzw. schraffierte Säulen: Darstellung der Variablen in Beziehung a) zur linken bzw. b) zur rechten Maßeinteilung. Durchschnittswerte +/-SEM
   * p< 0,0025,
   ** p< 0,01 versus SHAM

So liegt der Calciumgehalt der OR-X-Gruppe deutlich um ca. 50% niedrigerer als diejenigen von SHAM- und OR-X-CNC-Gruppe als Hinweis auf die Calciummobilisation aus dem Knochen. Der weitaus größte durch die erfindungsgemäße Verbindung hervorgerufene Calciumgehalt, direkt bezogen auf die Knochenascheanalyse, demonstriert, daß im Vergleich zu der Kontrolle deutlich mehr Calcium, nämlich ca. 10 bis 25%, eingebaut wurde, wohingegen verglichen mit der Gruppe von kastrierten Ratten ohne CNC-Zugabe (OR-X) sogar mehr als 30 % Calcium eingebaut wird.

Bei den OR-X-Ratten ergeben sich folglich auch niedrigere Werte beim Knochenvolumen und beim Trockengewicht. Bei den OR-X + CNC-Ratten dagegen ist nicht nur die Wiederherstellung der Werte wie bei den SHAM-Ratten zu erkennen sondern auch eine gegenüber der SHAM-Gruppe höhere Knochendichte festzustellen, wie dem unteren Teil der Abb. 5 zu entnehmen ist.

Das Molverhältnis von Calcium/Phosphor ist in Tab. 7 wiedergegeben.

**Tab. 7**

| **Calcium/Phosphor-Quotienten** | | |
|---|---|---|
| SHAM | OR-X | OR-X+CNC |
| 1.6±0 | 1.4±0.1, p<0.01 | 1.8±0.1 |

Die Werte der OR-X Gruppe weisen darauf hin, daß eine durch die Orchiektomie verursachte Reifungsstörung von Calciumphosphat zu Hydroxyapatit stattfindet. Im Gegensatz dazu normalisiert überraschenderweise die zusätzliche CNC-Zufuhr diese Variablen nicht nur, sondern es werden im Falle des Calcium/Phosphor-Quotienten, der Knochendichte und des Knochencalciums die Durchschnittswerte der SHAM-Ratten sogar eindrucksvollerweise wiederum überschritten.

Was das Verhalten des Organismus unter einer Behandlung mit den erfindungsgemäßen Verbindungen betrifft, so ist über eine etwaige Beeinflussung von Körpergewicht sowie Nahrungs- und Flüssigkeitshaushalt, Nierenfunktion, Citrat- und Mineralienhaushalt in Blut und Urin sowie Blut-pH-Werte bereits vorher referiert worden. Dadurch wird deutlich, daß dabei keine nachteiligen Stoffwechsel- oder sonstige Veränderungen eintreten; die beschriebenen Studien zeigen deutlich die Unbedenklichkeit der Verbindungen.

Die erforderlichen Ausgangssubstanzen zur Herstellung der Calcium-Alkalimetallcitrate wie Citronensäure, Kalium-oder Natriumcarbonat und Calciumcitrat sind chemische Massenprodukte und somit preiswerte Grundstoffe, so daß auch das Erfordernis der kostengünstigen Behandlung erfüllt ist.

## Patentansprüche

1. Pharmazeutisches Mittel, **dadurch gekennzeichnet,** daß das pharmazeutische Mittel ein oder mehrere Calcium-Alkalimetallcitrate der Formel CaXC₆H₅O₇, worin X ein Alkalimetall ist, enthält.

2. Pharmazeutisches Mittel nach Anspruch 1, **dadurch gekennzeichnet,** daß das Alkalimetall Natrium oder Kalium ist.

3. Pharmazeutisches Mittel nach Anspruch 1 oder 2, **dadurch gekennzeichnet,** daß das pharmazeutische Mittel Calcium-Alkalimetallcitrate als Hydrat oder Hydratgemisch enthält.

4. Pharmazeutisches Mittel nach einem der Ansprüche 1 bis 3, **dadurch gekennzeichnet,** daß das pharmazeutische Mittel einen oder mehrere Zusatzstoffe enthält.

5. Pharmazeutisches Mittel nach Anspruch 4, **dadurch gekennzeichnet,** daß der Zusatzstoff Citronensäure ist.

6. Verfahren zur Herstellung der Verbindung nach Anspruch 1, **dadurch gekennzeichnet,** daß man eine wäßrige Ca-Citrat-Suspension mit einem Alkalimetallcitrat und Citronensäure *in konzentrierter wäßriger Lösung* in einem Molverhältnis von Calciumcitrat, Alkalimetallcitrat und Citronensäure von 2:2:1 bis 2:2,5:1 bei einem *pH-Bereich von 4,6 bis 4,8* bei einer *Temperatur von 80 bis 95°C* unter Rückfluß während 1 bis 1,5 Stunden unter Rührung umsetzt und man entweder nach der Umsetzung die Lösung mit dem gebildeten Niederschlag filtriert, den Rückstand mindestens einmal mit Wasser wäscht und trocknet oder man nach der Umsetzung die Lösung mit dem gebildeten Niederschlag sofort bis zur Trockne einengt, oder daß nach der Umsetzung die Lösung mit dem gebildeten Niederschlag als Suspension das Endprodukt ist.

7. Verfahren nach Anspruch 6, **dadurch gekennzeichnet,** daß man eine wäßrige Suspension aus Calciumcitrat-Suspension mit 31,7 % (w/v) Calciumcitrat^{.}4H₂O mit einer wäßrigen Lösung aus 0,56 bis 0,7 m Alkalimetallcitrat und 0,28 m Citronensäure umsetzt.

8. Verfahren zur Herstellung der Verbindungen nach Anspruch 1, **dadurch gekennzeichnet,** daß man entweder Calciumcitrat mit einem Alkalimetallcarbonat und Citronensäure *in konzentrierter wäßriger Lösung* in einem Molverhältnis von Calciumcitrat, Alkalimetallcarbonat und Citronensäure von 2:3:3 bis 2:3,75:3 oder Calciumcitrat mit einem Alkalimetallhydrogencarbonat oder -hydroxid und Citronensäure in konzentrierter wäßriger Lösung in einem Molverhältnis von Calciumcitrat, Alkalimetallhydrogencarbonat bzw.-hydroxid und Citronensäure von 2:6:3 bis 2:7,5:3 bei einem *pH-Bereich von 4,6 bis 4, 8* bei einer Temperatur von *80 bis 95 °C* unter Rückfluß während 1 bis 1,5 Stunden unter Rührung umsetzt und man entweder nach der Umsetzung die Lösung mit dem Niederschlag filtriert, den Rückstand mindestens einmal mit Wasser wäscht und trocknet, oder man nach der Umsetzung die Lösung mit dem gebildeten Niederschlag sofort bis zur Trockne einengt, oder daß nach der Umsetzung die Lösung mit dem gebildeten Niederschlag als Suspension das Endprodukt ist.

9. Verfahren nach Anspruch 8, **dadurch gekennzeichnet,** daß man eine wäßrige Suspension aus 31,7 % (w/v) Calciumcitrat^{.}4H₂O mit einer wäßrigen Lösung von 0,83 m Citronensäure versetzt und unter Wasserzugabe Alkalimetallcarbonat bis zu einer Konzentration von 0,28 bis 0,35 m zugibt.

10. Verfahren zur Herstellung der Verbindungen nach Anspruch 1, **dadurch gekennzeichnet,** daß man Calciumcarbonat oder -oxid mit einem Alkalimetallcarbonat und Citronensäure i*n konzentrierter wäßriger Lösung* in einem Molverhältnis Calciumcarbonat, Alkalimetallcarbonat und Citronensäure von 6:3:7 bis 6:3,75:7 bei einem *pH-Bereich von 4,6 bis 4,8* bei einer Temperatur von *80 bis 95 °C* unter Rückfluß während 1 bis 1,5 Stunden unter Rührung umsetzt und man entweder nach der Umsetzung die Lösung mit dem Niederschlag filtriert, den Rückstand mindestens einmal mit Wasser wäscht und trocknet, oder man nach der Umsetzung die Lösung mit dem gebildeten Niederschlag sofort bis zur Trockne einengt, oder daß nach der Umsetzung die Lösung mit dem gebildeten Niederschlag als Suspension das Endprodukt ist.

11. Verfahren nach Anspruch 10, **dadurch gekennzeichnet,** daß man einer 1,89 m wäßrigen Citronensäure-Lösung unter Rühren Calciumcarbonat in einer solchen Menge zugibt, daß eine Konzentration von 1,62 m Calciumcarbonat vorliegt und Alkalimetallcarbonat bis zu einer Konzentration von 0,54 bis 0,68 m zusetzt.

12. Verfahren nach einem der Ansprüche 6 bis 11, **dadurch gekennzeichnet,** daß das Alkalimetallcarbonat Natrium- oder Kaliumcarbonat ist.

13. Verfahren nach einem der Ansprüche 6 bis 12, **dadurch gekennzeichnet,** daß das Alkalimetallhydrogencarbonat Natrium- oder Kaliumhydrogencarbonat ist.

14. Verfahren nach einem der Ansprüche 6 bis 13, **dadurch gekennzeichnet,** daß man nach der Waschung des Rückstandes Citronensäure im Überschuß zu dem Rückstand gibt.

15. Verwendung von mindestens einem Calcium-Alkalimetallcitrat der Formel CaXC₆H₅O₇, worin X ein Alkalimetall ist, oder eines Hydrats oder Hydratgemisches davon zur Herstellung eines Arzneimittels für die therapeutische Verwendung zum Aufbau von Muskelproteinen und zur Erhöhung der muskulären Stoffwechselleistung durch antikatabolische Wirkung.

16. Verwendung von mindestens einem Calcium-Alkalimetallcitrat der Formel CaXC₆H₅O₇, worin X ein Alkalimetall ist, oder eines Hydrats oder Hydratgemisches davon zur Herstellung eines Arzneimittels für die therapeutische Verwendung zur Rekonvaleszenz oder palliativen Behandlung bei kataboler Stoffwechsellage chronisch konsumierender oder degenerativer Krankheiten.

17. Verwendung nach Anspruch 16 zur Verringerung des Proteinabbaus bei Tumorkachexie, Niereninsuffizienz, seniler Kachexie, Alkoholabusus und Infekten.

18. Verwendung nach Anspruch 16, bei Strahlen- oder Zytostatikatherapie, verlängerter Kortikoidtherapie, postoperativen oder posttraumatischen Zuständen.

19. Verwendung von mindestens einer Calcium-Alkalimetallcitrat-Verbindung der allgemeinen Formel CaXC₆H₅O₇, worin X ein Alkalimetall ist oder eines Hydrats oder Hydratgemisches davon, zur Herstellung eines Arzneimittels für die therapeutische Verwendung bei der Prophylaxe oder Behandlung des Knochengewebeschwundes und bei der Verstärkung der Knochenstrukturelemente.

20. Verwendung von mindestens einer Calcium-Alkalimetallcitrat-Verbindung der allgemeinen Formel CaXC₆H₅O₇, worin X ein Alkalimetall ist oder eines Hydrats oder Hydratgemisches davon, zur Herstellung eines Arzneimittels für die therapeutische Verwendung bei der Prophylaxe oder Behandlung der Osteoporose.

21. Verwendung nach einem der Ansprüche 15 bis 20, **dadurch gekennzeichnet,** daß das Calcium-Alkalimetallcitrat in Form einer Mischung mit ein oder mehreren Zusatzstoffen verabreicht wird.

22. Verwendung nach einem der Ansprüche 15 bis 20, **dadurch gekennzeichnet,** daß als Zusatzstoff Citronensäure verabreicht wird.

23. Verwendung nach einem der Ansprüche 15 bis 20, **dadurch gekennzeichnet,** daß als Calcium-Alkalimetallcitrat CaNaC₆H₅O₇ oder CaKC₆H₅O₇ verabreicht wird.

## Claims

1. Pharmaceutical agent, characterised in that the pharmaceutical agent contains one or more calcium-alkali metal citrates corresponding to the formula CaXC₆H₅O₇, wherein X is an alkali metal.

2. Pharmaceutical agent according to claim 1, characterised in that the alkali metal is sodium or potassium.

3. Pharmaceutical agent according to claim 1 or 2, characterised in that the pharmaceutical agent contains calcium-alkali metal citrates as hydrate or hydrate mixture.

4. Pharmaceutical agent according to one of claims 1 to 3, characterised in that the pharmaceutical agent contains one or more additives.

5. Pharmaceutical agent according to claim 4, characterised in that the additive is citric acid.

6. Process for the preparation of the compound according to claim 1, characterised in that an aqueous Ca citrate suspension is reacted with an alkali metal citrate and citric acid *in concentrated aqueous solution* in a molar ratio of calcium citrate, alkali metal citrate and citric acid of from 2:2:1 to 2:2.5:1 at a *pH range of 4.6 to 4.8* at a *temperature of 80°C to 95°C* under reflux for 1 to 1.5 hours, with stirring, and either after the reaction the solution containing the precipitate formed is filtered, the residue is washed at least once with water and dried, or after the reaction the solution containing the precipitate formed is immediately evaporated to dryness, or that after the reaction the solution containing the precipitate formed as suspension is the end product.

7. Process according to claim 6, characterised in that an aqueous suspension of calcium citrate suspension containing 31.7% (w/v) calcium citrate·4H₂O is reacted with an aqueous solution of 0.56 to 0.7 m alkali metal citrate and 0.28 m citric acid.

8. Process for the preparation of the compounds according to claim 1, characterised in that either calcium citrate is reacted with an alkali metal carbonate and citric acid *in concentrated aqueous solution* in a molar ratio of calcium citrate, alkali metal carbonate and citric acid of from 2:3:3 to 2:3.75:3, or calcium citrate is reacted with an alkali metal hydrogen carbonate or alkali metal hydroxide and citric acid in concentrated aqueous solution in a molar ratio of calcium citrate, alkali metal hydrogen carbonate or alkali metal hydroxide and citric acid of from 2:6:3 to 2:7.5:3 at a *pH range of 4.6 to 4.8* at a temperature of *80°C to 95°C* under reflux for 1 to 1.5 hours, with stirring, and either after the reaction the solution containing the precipitate is filtered, the residue is washed at least once with water and dried, or after the reaction the solution containing the precipitate formed is immediately evaporated to dryness, or that after the reaction the solution containing the precipitate formed as suspension is the end product.

9. Process according to claim 8, characterised in that an aqueous solution of 0.83 m citric acid is added to an aqueous suspension of 31.7% (w/v) calcium citrate·4H₂O and, with the addition of water, alkali metal carbonate is added thereto up to a concentration of 0.28 to 0.35 m.

10. Process for the preparation of the compounds according to claim 1, characterised in that calcium carbonate or calcium oxide is reacted with an alkali metal carbonate and citric acid *in concentrated aqueous solution* in a molar ratio of calcium carbonate, alkali metal carbonate and citric acid of from 6:3:7 to 6:3.75:7 at a *pH range of 4.6 to 4.8* at a temperature of *80°C to 95°C* under reflux for 1 to 1.5 hours, with stirring, and either after the reaction the solution containing the precipitate is filtered, the residue is washed at least once with water and dried, or after the reaction the solution containing the precipitate formed is immediately evaporated to dryness, or that after the reaction the solution containing the precipitate formed as suspension is the end product.

11. Process according to claim 10, characterised in that calcium carbonate is added, with stirring, to a 1.89 m aqueous citric acid solution, in a quantity such that calcium carbonate is present in a concentration of 1.62 m and alkali metal carbonate is added thereto up to a concentration of 0.54 to 0.68 m.

12. Process according to one of claims 6 to 11, characterised in that the alkali metal carbonate is sodium carbonate or potassium carbonate.

13. Process according to one of claims 6 to 12, characterised in that the alkali metal hydrogen carbonate is sodium hydrogen carbonate or potassium hydrogen carbonate.

14. Process according to one of claims 6 to 13, characterised in that after the washing of the residue, citric acid is added in excess to the residue.

15. Use of at least one calcium-alkali metal citrate corresponding to the formula CaXC₆H₅O₇, wherein X is an alkali metal, or of a hydrate or hydrate mixture thereof, for the manufacture of a medicament for therapeutic use for the anabolism of muscle proteins and for increasing muscular metabolic efficiency by anticatabolic action.

16. Use of at least one calcium-alkali metal citrate corresponding to the formula CaXC₆H₅O₇, wherein X is an alkali metal, or of a hydrate or hydrate mixture thereof, for the manufacture of a medicament for therapeutic use for reconvalescence or palliative treatment of chronic consumptive or degenerative diseases in catabolic metabolic situations.

17. Use according to claim 16 for decreasing proteolysis in tumour-induced cachexia, renal insufficiency, senile cachexia, alcohol abuse and infections.

18. Use according to claim 16 in radiation therapy or cytostatic therapy, prolonged corticoid therapy, postoperative or post-traumatic conditions.

19. Use of at least one calcium-alkali metal citrate compound corresponding to the general formula CaXC₆H₅O₇, wherein X is an alkali metal, or of a hydrate or hydrate mixture thereof, for the manufacture of a medicament for therapeutic use in the prophylaxis or treatment of loss of bone tissue and for increasing the structural elements of bones.

20. Use of at least one calcium-alkali metal citrate compound corresponding to the general formula CaXC₆H₅O₇, wherein X is an alkali metal, or of a hydrate or hydrate mixture thereof, for the manufacture of a medicament for therapeutic use in the prophylaxis or treatment of osteoporosis.

21. Use according to one of claims 15 to 20, characterised in that the calcium-alkali metal citrate is administered in the form of a mixture containing one or more additives.

22. Use according to one of claims 15 to 20, characterised in that citric acid is administered as additive.

23. Use according to one of claims 15 to 20, characterised in that the calcium-alkali metal citrate administered is CaNaC₆H₅O₇ or CaKC₆H₅O₇.

## Revendications

1. Composition pharmaceutique, caractérisée en ce que la composition pharmaceutique contient un ou plusieurs citrates de calcium et d'un métal alcalin, de formule CaXC₆H₅O₇ dans laquelle X est un métal alcalin.

2. Composition pharmaceutique selon la revendication 1, caractérisée en ce que le métal alcalin est le sodium ou le potassium.

3. Composition pharmaceutique selon la revendication 1 ou 2, caractérisée en ce que la composition pharmaceutique contient des citrates de calcium et d'un métal alcalin sous forme d'hydrates ou de mélanges d'hydrates.

4. Composition pharmaceutique selon l'une des revendications 1 à 3, caractérisée en ce que la composition pharmaceutique contient un ou plusieurs additifs.

5. Composition pharmaceutique selon la revendication 4, caractérisée en ce que l'additif est l'acide citrique.

6. Procédé de préparation du composé selon la revendication 1, caractérisé en ce que l'on fait réagir une suspension aqueuse de citrate de Ca avec un citrate de métal alcalin et de l'acide citrique *en solution aqueuse concentrée* dans un rapport molaire du citrate de calcium, du citrate de métal alcalin et de l'acide citrique compris entre 2:2:1 et 2:2,5:1, à un *pH compris entre 4,6 et 4,8,* à une *température de 80 à 95°C,* au reflux, pendant 1 à 1,5 heure en agitant, et, soit on filtre la solution avec le précipité formé après la réaction, on lave le résidu au moins une fois avec de l'eau et on le sèche, soit on concentre tout de suite à siccité la solution avec le précipité formé après la réaction, soit la solution avec le précipité formé après la réaction constitue le produit final sous forme de suspension.

7. Procédé selon la revendication 6, caractérisé en ce que l'on fait réagir une suspension aqueuse constituée d'une suspension de citrate de calcium à 31,7 % (m/v) de citrate de calcium • 4 H₂O avec une solution aqueuse de citrate de métal alcalin 0,56 à 0,7 M et d'acide citrique 0,28 M.

8. Procédé de préparation des composés selon la revendication 1, caractérisé en ce que l'on fait réagir du citrate de calcium avec un carbonate de métal alcalin et de l'acide citrique *en solution aqueuse concentrée* dans un rapport molaire du citrate de calcium, du carbonate de métal alcalin et de l'acide citrique de 2:3:3 à 2:3,75:3, ou du citrate de calcium avec un hydrogénocarbonate ou un hydroxyde de métal alcalin et de l'acide citrique en solution aqueuse concentrée dans un rapport molaire du citrate de calcium, de l'hydrogénocarbonate ou de l'hydroxyde de métal alcalin et de l'acide citrique de 2:6:3 à 2:7,5:3 à un *pH compris entre 4,6 et 4,8,* à une *température de 80 à 95°C,* au reflux, pendant 1 à 1,5 heure en agitant, et, soit on filtre la solution avec le précipité après la réaction, on lave le résidu au moins une fois avec de l'eau et on le sèche, soit on concentre tout de suite à siccité la solution avec le précipité formé après la réaction, soit la solution avec le précipité formé après la réaction constitue le produit final sous forme de suspension.

9. Procédé selon la revendication 8, caractérisé en ce que l'on ajoute une solution aqueuse d'acide citrique 0,83 M à une suspension aqueuse de 31,7 % (m/v) de citrate de calcium • 4 H₂O, et, en ajoutant de l'eau, on ajoute du carbonate de métal alcalin jusqu'à une concentration de 0,28 à 0,35 M.

10. Procédé de préparation des composés selon la revendication 1, caractérisé en ce que l'on fait réagir du carbonate ou de l'oxyde de calcium avec un carbonate de métal alcalin et de l'acide citrique *en solution aqueuse concentrée* dans un rapport molaire du carbonate de calcium, du carbonate de métal alcalin et de l'acide citrique de 6:3;7 à 6:3,75:7, à un *pH compris entre 4,6 et 4,8,* à une *température de 80 à 95°C,* au reflux, pendant 1 à 1,5 heure en agitant, et, soit on filtre la solution avec le précipité après la réaction, on lave le résidu au moins une fois avec de l'eau et on le sèche, soit on concentre tout de suite à siccité la solution avec le précipité formé après la réaction, soit la solution avec le précipité formé après la réaction constitue le produit final sous forme de suspension.

11. Procédé selon la revendication 10, caractérisé en ce que l'on ajoute à une solution aqueuse d'acide citrique 1,89 M, en agitant, du carbonate de calcium en une quantité telle que l'on obtient une concentration de carbonate de calcium de 1,62 M, et on ajoute du carbonate de métal alcalin jusqu'à une concentration de 0,54 à 0,68 M.

12. Procédé selon l'une des revendications 6 à 11, caractérisé en ce que le carbonate de métal alcalin est du carbonate de sodium ou de potassium.

13. Procédé selon l'une des revendications 6 à 12, caractérisé en ce que l'hydrogénocarbonate de métal alcalin est l'hydrogénocarbonate de sodium ou de potassium.

14. Procédé selon l'une des revendications 6 à 13, caractérisé en ce que, après avoir lavé le résidu, on ajoute de l'acide citrique en excès au résidu.

15. Utilisation d'au moins un citrate de calcium et de métal alcalin de formule CaXC₆H₅O₇, dans laquelle X est un métal alcalin, ou d'un de ses hydrates ou mélanges d'hydrates, pour la préparation d'un médicament destiné à être utilisé en thérapeutique pour la construction de protéines musculaires et pour augmenter la puissance du métabolisme musculaire par une action anticatabolique.

16. Utilisation d'au moins un citrate de calcium et de métal alcalin de formule CaXC₆H₅O₇, dans laquelle X est un métal alcalin, ou d'un de ses hydrates ou mélanges d'hydrates, pour la préparation d'un médicament destiné à être utilisé en thérapeutique pour la convalescence ou le traitement palliatif dans une situation de métabolisme catabolique de maladies avec consommation chronique ou dégénératives.

17. Utilisation selon la revendication 16, destinée à la réduction de la dégradation des protéines dans le cas d'une cachexie tumorale, d'une insuffisance rénale, d'une cachexie sénile, d'un abus d'alcool et d'infections.

18. Utilisation selon la revendication 16, dans le cas d'une radiothérapie ou d'une thérapie par des cytostatiques, d'une corticothérapie prolongée, d'états postopératoires ou post-traumatiques.

19. Utilisation d'au moins un citrate de calcium et de métal alcalin de formule générale CaXC₆H₅O₇, dans laquelle X est un métal alcalin, ou d'un de ses hydrates ou mélanges d'hydrates, pour la préparation d'un médicament destiné à être utilisé en thérapeutique dans la prophylaxie ou le traitement de la diminution des tissus osseux et pour renforcer les éléments structurels des os.

20. Utilisation d'au moins un citrate de calcium et de métal alcalin de formule générale CaXC₆H₅O₇, dans laquelle X est un métal alcalin, ou d'un de ses hydrates ou mélanges d'hydrates, pour la préparation d'un médicament destiné à être utilisé en thérapeutique dans la prophylaxie ou le traitement de l'ostéoporose.

21. Utilisation selon l'une des revendications 15 à 20, caractérisée en ce que l'on administre le citrate de calcium et de métal alcalin sous forme d'un mélange avec un ou plusieurs additifs.

22. Utilisation selon l'une des revendications 15 à 20, caractérisée en ce que l'on administre de l'acide citrique comme additif.

23. Utilisation selon l'une des revendications 15 à 20, caractérisée en ce que l'on administre comme citrate de calcium et de métal alcalin du CaNaC₆H₅O₇ ou du CaKC₆H₅O₇.
